(19) Europäisches Patentamt
European Patent Office
Office européen des brevets

(11) Numéro de publication : **0 514 268 A1**

# (12) DEMANDE DE BREVET EUROPEEN

(21) Numéro de dépôt : **92401318.8**

(22) Date de dépôt : **14.05.92**

(51) Int. Cl.$^5$ : **C07D 471/04,** C07D 401/04, C07D 205/04, C07D 215/56, A61K 31/47

(30) Priorité : **16.05.91 FR 9105937**

(43) Date de publication de la demande : **19.11.92 Bulletin 92/47**

(84) Etats contractants désignés : **AT BE CH DE DK FR GB GR IT LI LU MC NL PT SE**

(71) Demandeur : **LABORATORIOS DEL DR. ESTEVE, S.A.
Av. Mare de Deu de Montserrat, 221
E-08026 Barcelona (ES)**

(72) Inventeur : **Corbera-Arjona, Jordi
Miquel Vives, 95, atico D
E-08222 Tarrasa, Barcelone (ES)**
Inventeur : **Frigola-Constansa, Jordi
Av. Diagonal, 299 at. la
E-08013 Barcelone (ES)**
Inventeur : **Pares-Corominas, Juan
Padilla, 349, 3o 3a
E-08025 Barcelone (ES)**

(74) Mandataire : **Ahner, Francis
CABINET REGIMBEAU, 26, avenue Kléber
F-75116 Paris (FR)**

(54) **Dérivés de pyridone amino acide azétidinyl substitués, leur préparation et leur application en tant que médicaments.**

(57) La présente invention concerne les composés répondant à la formule générale I

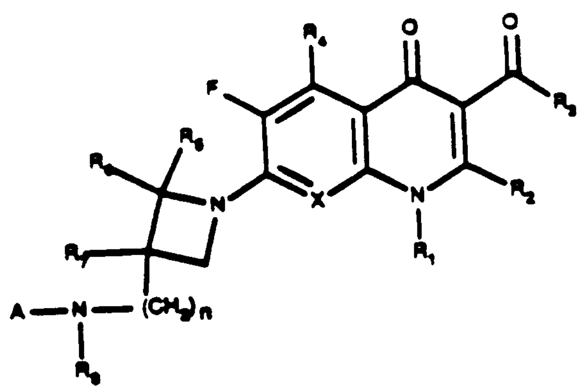

I

et leurs sels pharmaceutiquement acceptables, formule dans laquelle

x représente un atome d'azote ou bien un atome de carbone lié à un atome d'hydrogène, lié à un atome d'halogène, un radical hydroxy ou alkyloxy, un radical alkyle, un radical alkyle halogéné, un radical alkylamino ;

$R_1$ représente un radical alkyle ou cycloalkyle inférieur, un radical halogénoalkyle inférieur, un radical hydroxyalkyle, un radical vinyle un radical aryle ou un radical aryle substitué par un ou plusieurs atomes de fluor, ou un radical alkylamino ;

$R_2$ représente un atome d'hydrogène ou un radical alkyle inférieur ;

$R_3$ représente un radical hydroxy ou un radical alkyloxy inférieur de $C_1$ à $C_4$ ;

$R_4$ représente un atome d'hydrogène, un atome de fluor, un radical alkyl inférieur, un radical nitro, un radical amino ou un radial amino substitué ;

$R_5$, $R_6$ et $R_7$ représentent un atome d'hydrogène ou un radical alkyle inférieur ;

n est un nombre qui peut être 0 ou 1 ;

X et $R_1$ peuvent former ensemble une liaison représentée par un groupe $C-CH_2-CH_2-CH-R_9$ou $C-O-CH-CH_2-CH-R_9$ ou $C-S-CH_2-CH-R_9$ dans lesquels $R_9$ représente un atome d'hydrogène, un radical alkyle inférieur ou un radical halogénoalkyle inférieur.

EP 0 514 268 A1

$R_1$ et $R_2$ peuvent former ensemble une liaison représentée par un groupe $-CHR_{10}-S-$ dans lequel $R_{10}$ représente un atome d'hydrogène ou un radical alkyl inférieur ou un radical halogénoalkyle inférieur ;

$R_2$ et $R_3$ peuvent former ensemble une liaison représentée par un groupe -S-NH- ;

A est un groupe solubilisateur qui est représenté par un aminoacide résiduel ou une chaine polypeptidique constituée de deux à quatre ou plus résidus d'aminoacides, lesquels sont liés de façon covalente entre eux par des liaisons peptidiques, et leur procédé de préparation.

La présente invention décrit des nouveaux dérivés azétidiniques des acides pyridonecarboxyliques tels que 1,4-dihydro-4-oxoquinoléine-3-carboxylique, 1,8-naphtyridine-4-oxo-3-carboxylique et 2,3-dihydro-7-oxo-7H-pyrido-[1,2,3-de]-1,4-benzoxazine-6-carboxylique, ainsi que des dérivés azétidiniques d'isothiazolopyridone tels que 2,3,4,9-tétrahydroisothiazolo[5,4-b]naphtyridine-3,4-dione, 1,2,8,9-tétrahydro-7H-isothiazolo[4′,5′ :5,6]-pyrido[1,2, 3-de]-benzoxazine-7,8-dione, les sels thérapeutiquement acceptables de ces composés, leur procédé de préparation, ainsi que leur application comme médicaments.

La présente invention est une suite de la demande de brevet européen 88403352.3, où l'on décrit des dérivés azétidiniques de quinolones et de pyridobenzoxazines. C'est également une suite de la demande de brevet européen 90400684.8 où l'on décrit différents dérivés azétidiniques de naphtyridines, de quinolones de pyridobenzoxazines. C'est encore également une suite de la demande de brevet européen 90401036.0 où l'on décrit différents dérivés azétidiniques d'isothiazolonaphtyridines, d'isothiazoloquinolones et d'isothiazolol-pyrido-benzoxazines, tous étant des composés possédant une très bonne activité antimicrobienne, mais qui nonobstant possèdent une faible solubilité à pH physiologique, ce qui réduit leur absorption par voie orale et possèdent également une faible solubilité dans l'eau, ce qui rend difficile leur emploi dans des formulations injectables pour application par voie intraveineuse, intramusculaire ou souscutanée.

La présente invention décrit des nouveaux dérivés azétidiniques de quinolones, de naphtyridines, de pyridobenzoxazines, d'isothiazolo-quinolones, d'isothiazolonaphtyridines et d'isothiazolopyrido-benzoxazines dans lesquels l'anneau d'azétidine est 3-A-aminoazétidine ou bien 3-A-aminométhylazétidine dans lesquelles A est un groupe solubilisateur. Le groupe solubilisateur A est un résidu d'aminoacide ou une chaîne polypeptidique, de préférence un di-, tri- ou tétrapeptide, ou bien ces mêmes aminoacides ou chaînes polypeptidiques dans lesquels les groupes fonctionnels sont protégés convenablement par des groupes tels qu'un benzyloxycarbonyl, un tert-butoxycarbonyl, des éthers comme par exemple un benzyléther ou un tert-butyléther, le N-trifluoroacétyl ou d'autres groupes protecteurs bien connus pour la synthèse de peptides, ainsi que leurs sels thérapeutiquement acceptables.

Les composés objets de la présente invention répondent à la formule générale I

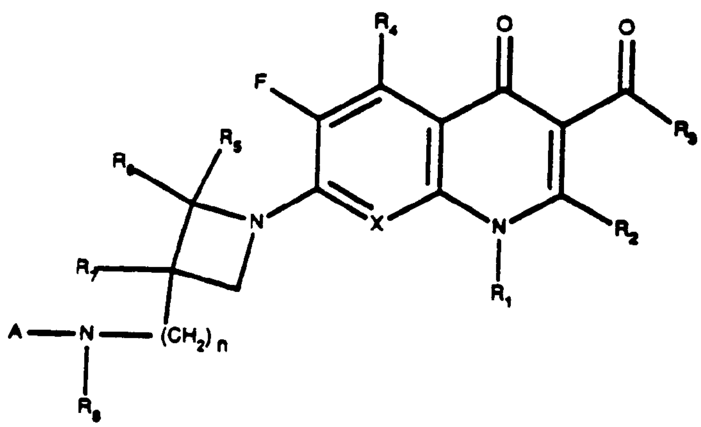

I

dans laquelle

X représente un atome d'azote ou bien un atome de carbone lié à un atome d'hydrogène (C-H) ou bien un atome de carbone lié à un atome d'halogène (C-Y) dans lequel Y représente un atome de fluor, de chlore ou de brome, ou bien un atome de carbone lié à un radical hydroxy (C-OH) ou alkyloxy (C-O-alkyl), ou bien un atome de carbone lié à un radical alkyle (C-alkyl), ou bien un atome de carbone lié à un radical alkyle halogéné ($C-CF_3$), ou bien un atome de carbone lié à un radical alkylamino (C-NH-alkyle).

$R_1$ représente un radical alkyle ou cycloalkyle inférieur, un radical halogénoalkyle inférieur, un radical hydroxyalkyle, un radical vinyle, un radical aryle ou un radical aryle substitué par un ou plus atomes de fluor, ou un radical alkylamino (NH-alkyle).

$R_2$ représente un atome d'hydrogène ou un radical alkyle inférieur.

$R_3$ représente un radical hydroxy ou un radical alkyloxy inférieur de $C_1$ à $C_4$.

$R_4$ représente un atome d'hydrogène, un atome de fluor, un radical alkyl inférieur, un radical nitro, un radical amino ou un radical amino substitué.

$R_5$, $R_6$ et $R_7$ représentent un atome d'hydrogène ou un radical alkyl inférieur. Les substituants azétidiniques

peuvent avoir selon la nature et la position relative, jusqu'à deux centres chiraux, chacun d'eux avec une configuration "R" ou "S".

$R_8$ représente un atome d'hydrogène ou un radical alkylé inférieur.

n est un nombre qui peut être 0 ou 1.

X et $R_1$ peuvent former ensemble une liaison représentée par un groupe C-$CH_2$-$CH_2$-CH-$R_9$ ou C-O-$CH_2$-CH-$R_9$ ou C-S-$CH_2$-CH-$R_9$ dans lesquels $R_9$ représente un atome d'hydrogène, un radical alkyle inférieur ou un radical halogénoalkyle inférieur et possédant dans ces deux derniers cas un centre chiral avec une configuration "R" ou "S".

$R_1$ et $R_2$ peuvent former ensemble une liaison représentée par un groupe -$CHR_{10}$-S- dans lequel $R_{10}$ représente un atome d'hydrogène ou un radical alkyle inférieur ou un radical halogénoalkyle inférieur et possédant dans ces deux derniers cas un centre chiral avec une configuration "R" ou "S".

$R_2$ et $R_3$ peuvent former ensemble une liaison représentée par un groupe -S-NH-.

A est un groupe solubilisateur qui est représenté par un aminoacide résiduel ou une chaîne polypeptidique constituée de deux à quatre ou plus résidus d'aminoacides, lesquels sont liés de façon covalente entre eux à travers des liaisons peptidiques, ainsi que leurs sels pharmaceutiquement acceptables.

Les résidus d'aminoacides de la présente invention comprennent les 20 aminoacides naturels, désignés moyennant leurs trois lettres symboliques, ainsi que d'autres aminoacides tel que la norvaline (Nva), homosérine, 4-hydroxyproline, hydroxylysine, ornitine, etc.

Par convention, les désignations symboliques des aminoacides sont les suivantes :

| | |
|---|---|
| Alanine | Ala |
| Arginine | Arg |
| Asparagine | Asn |
| Ac. Aspartique | Asp |
| Ac. Glutamique | Glu |
| Cystéīne | Cys |
| Glutamine | Glu |
| Glycine | Gly |
| Histidine | His |
| Isoleucine | Ile |
| Leucine | Leu |
| Lysine | Lys |
| Metionine | Met |
| Phénylalanine | Phe |
| Proline | Pro |
| Sérine | Ser |
| Thréonine | Thr |
| Tryptophane | Trp |
| Tyrosine | Tyr |
| Valine | Val |

On doit signaler que le groupe 3-amino ou 3-aminométhyl du cycle d'azétidine forme une liaison avec le groupe carboxylique du résidu de l'aminoacide A. Dans le cas des chaînes polypeptidiques, les résidus d'aminoacides sont liés entre eux moyennant une liaison peptidique conventionnelle, ainsi le groupe alpha-amino du premier résidu d'aminoacide lié est lié au groupe carboxylique du second résidu d'alpha-aminoacide, etc.

La configuration stéréochimique des aminoacides de la présente invention peuvent être de la forme D- ou L- ou un mélange des deux, étant préférable, nonobstant, l'utilisation de résidus d'aminoacides sous forme L-.

Les composés, objets de la présente invention, sont particulièrement actifs face à des microorganismes bactériens et par conséquent ils sont utiles en médecine humaine ou vétérinaire pour la prophylaxie et la chimiothérapie des infections locales et systémiques produites par des pathogènes aérobies ou anaérobies, tant Gram-positifs comme Gram-négatifs. En plus de présenter une activité antibactérienne élevée, les composés objets de la présente invention montrent une augmentation de la solubilité tant à pH physiologique que dans l'eau ce qui permet leur utilisation par voie parentérale, ainsi qu'une excellente biodisponibilité. D'autre part, les composés objets de la présente invention peuvent agir comme pro-drogues des azétidinylquinolones, azétidinylnaphtyridines, azétidinylpyridobenzoxazines, azétidinylisothiazolo-quinolones, azétidinylisothiazolonaphtyridines ou azétidinylisothiazolopyrido-benzoxazines préalablement connus, étant donné que le groupe solubilisateur A, lorsque A est un résidu d'aminoacide ou une chaîne polypeptidique, peut subir une hydrolyse enzymatique qui produit la libération des azétidinylquinolones, des azétidinylnaphtyridines, des azétidinylpyridobenzoxazines, des azétidinylisothiazolo-quinolones, des azétidinylisothiazolopyrido-benzoxazines préalablement connus.

Les composés de formule générale I et leurs sels physiologiquement acceptables, tels que les sels d'acides minéraux comme par exemple les chlorhydrates, ainsi que les sels d'acides organiques comme par exemple les toluènesulfonates ou les méthylsulfonates, sont de préférence administrés sous cette forme dans les compositions pharmaceutiques.

SYNTHESE DES COMPOSES

Les composés de formule générale II qui ont un groupe 3-amino-1-azétidinyl ou un groupe 3-aminométhyl-1-azétidinyl, tous deux différemment substitués, et qui sont liés à la position 7 de différents naphtyridines et quinolones, ou qui sont liés à la position 10 des pyrido-benzoxazines ou qui sont liés à la position 7 des isothiazolo-naphtyridines et isothiazoloquinolones, ou qui sont liés à la position 11 des isothiazolopyridobenzoxazines, qui l'utilisent pour préparer les composés hétérocycliques de la formule générale I, peuvent se préparer par des méthodes connues comme celles qui sont décrites dans les demandes de brevets européens 88403352.3, 90400684.8 et 90401036.0.

Les composés hétérocycliques de formule générale IV, que l'on peut utiliser comme produits de départ pour préparer les composés de formule générale II, peuvent se préparer par des méthodes connues dans la bibliographie, (H. Koga, A. Itoh, S. Murayama, S. Suzne et T. Irikura, J. Med. Chem., 1980, 23, 1358 ; H. Egawa, T. Miyamoto, A. Minamida, Y. Nishimura, H. Okada, H. Uno et J. Matsumoto, J. Med. Chem., 1984, 27, 1543 ; D.T.W. Chu, P.B. Fernandes, A.K. Claiborne, L. Shen et A.G. Pernet, Drugs Exptl. Chim. Res., 14 (6), 379, (1988).

Les composés de formule générale VIII, que l'on peut utiliser comme produits de départ pour préparer les composés de formule générale I, peuvent se préparer par des méthodes connues comme celles qui se décrivent dans la demande de brevet européen 90401860.3.

Un schéma général pour la préparation des composés de la présente invention de formule générale I, moyennant des techniques de couplage d'aminoacides, est représenté dans le schéma suivant et détaillé dans les exemples 2aA, 3aA, 4aA, 2aL, 3aL, 4aL, 2bA, 3bA, 4bA, 2bL, 3bL, 4bL, 2ca, 3ca, 4ca, 3da, 4da.

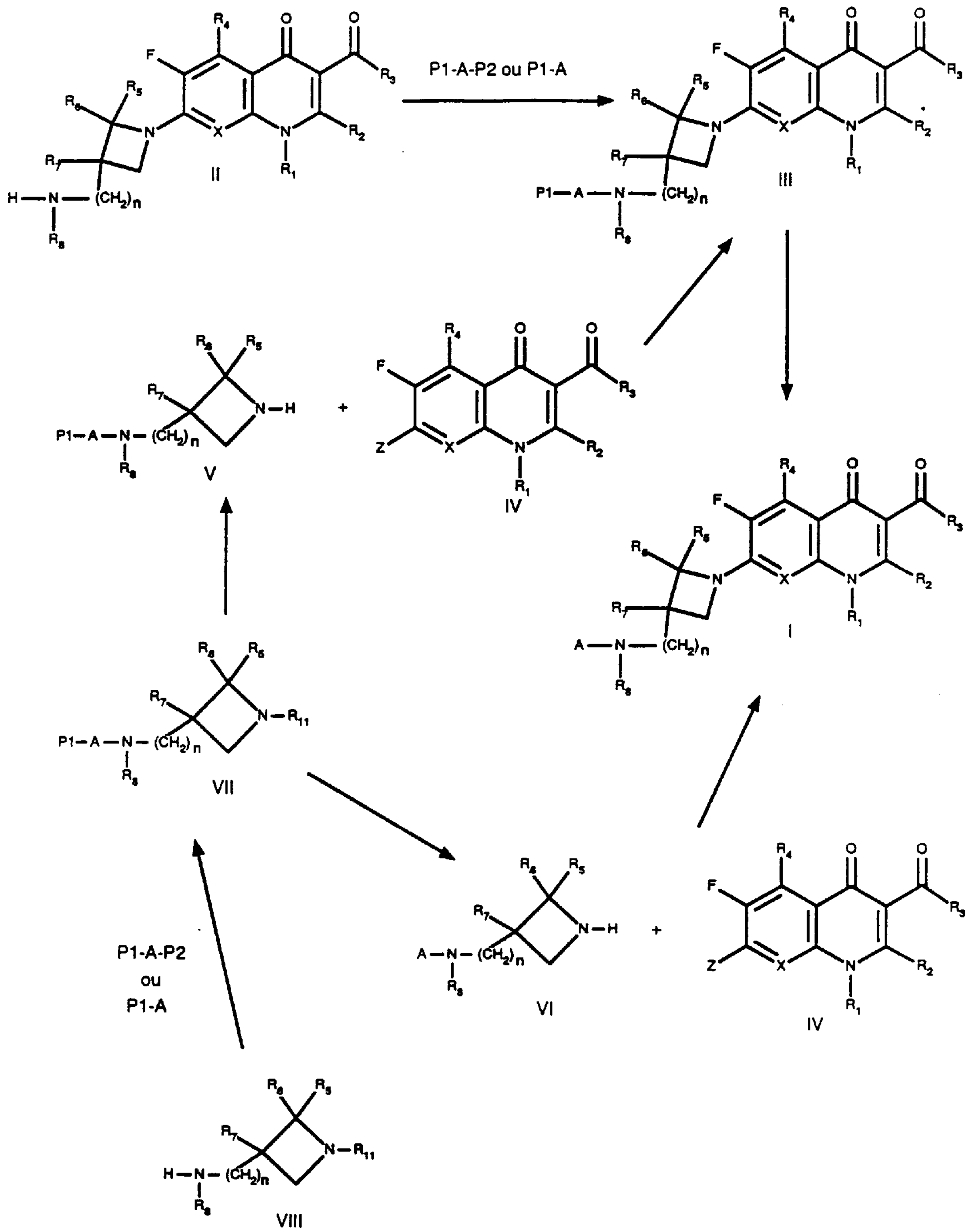
II
P1-A-P2 ou P1-A
III
V
IV
I
VII
VI
IV
P1-A-P2
ou
P1-A
VIII

Dans le schéma I, on représente différentes séquences de synthèses pour la préparation de composés de formule générale I dans laquelle A représente un résidu d'aminoacide.

SCHEMA I

Méthode A

Par réaction d'un composé de formule générale II, dans laquelle $R_1$, $R_2$, $R_3$, $R_4$, $R_5$, $R_6$, $R_7$, $R_8$, $R_9$, $R_{10}$, X et n ont les significations mentionnées précédemment, avec un composé de formule générale P1-A-P2, dans laquelle A a les significations mentionnées précédemment, et P1 représente n'importe lequel des groupes protecteurs de groupes fonctionnels utilisés dans la synthèse des peptides (M. Bodansky, Principles of Peptide Synthesis) (Reactivity and Structure : Concepts in Organic Chemistry, V.16) Ed. Springer Verlag, Berlin Heidelberg 1984. M. Bodansky and A. Bodansky, The practice of Peptide Synthesis (Reactivity and Structure : Concepts in Organic Chemistry, V.21), (Ed. Springer Verlag, Berlin Heidelberg 1984), tels que les protecteurs de la fonction amine comme par exemple benzyloxycarbonyl, tert-butoxycarbonyl, biphénylisopropyloxycarbonyl, 9-fluorénylméthyloxycarbonyl ainsi que d'autres groupes bien connus ; P1 représente aussi des groupes protecteurs de la chaîne tels que les éthers comme par exemple benzyléther, tert-butyléther, N-trifluoroacétyl, ou des protecteurs du groupe carboxylique tels que les esters comme par exemple benzyl esters ou tert-butyl esters, ainsi que d'autres groupes protecteurs bien connus (Theodora W. Greene, Protective Groups in Organic Chemistry, John Wiley and Sons, 1981) ; P 2 représente n'importe lequel des groupes activateurs de la fonction carboxylique utilisés dans la synthèse des peptides, comme par exemple le chlorure d'acide, un anhydride symétrique, un anhydride mixte tel que l'isovalérique ou le pivalique, ainsi que l'anhydride mixte du carbonate d'éthyle ou d'isobutyle, un ester activé tel que l'ester de N-hydroxysuccinimide, N-hydroxyphtalimide, cyanométhyle, O-nitrophényle, ainsi que l'emploi d'autres groupes activateurs de la fonction carboxylique bien connus, on obtient un composé de formule générale III.

Le composé de formule générale III peut se former également par réaction de II avec un composé de formule générale P1-A et avec l'aide de réactifs de couplage tels que DCC (dicyclohexylcarbodiimide), DCC en présence de 1-hydroxybenzotriazol, ou DCC en présence de N-hydroxysuccinimide, ou en utilisant IIDQ (1-isobutyloxycarbonyl-2-isobutyloxy-1,2-dihydroquinoline) ou EDC (N-éthyl-N'-3-diméthylaminopropylcarbodiimide chlorhydrate), ou d'autres réactifs bien connus pour la formation de liaisons peptidiques.

La réaction s'effectue en présence d'un solvant approprié, par exemple le diméthylformamide, le diméthylsulfoxyde, un alcool, un éther comme par exemple l'éther éthylique, le dioxane, le 1,2-diméthoxyéthane ou le tétrahydrofurane, l'acétate d'éthyle, le dichlorométhane, le chloroforme, le benzène, le toluène, la pyridine, ainsi qu'en milieu organo-aqueux comme la pyridine-eau.

La réaction a lieu en présence d'une base telle que la N-éthylmorpholine, N-méthylmorpholine, l'hydroxyde de sodium, la pyridine ou la triéthylamine.

La température de réaction varie entre -15°C et celle de reflux du solvant et le temps de réaction est compris entre dix minutes et 24 heures.

Méthode B

Par réaction d'un composé de formule générale III, dans laquelle $R_1$, $R_2$, $R_3$, $R_4$, $R_6$, $R_6$, $R_7$, $R_8$, $R_9$, $R_{10}$, X, A, P1 et n ont les significations mentionnées précédemment avec des réactifs utiles pour l'élimination de groupes protecteurs de groupes fonctionnels présents dans les aminoacides et peptides du composé de formule générale III, on obtient la préparation du composé de formule générale I.

Cette élimination des groupes protecteurs peut se réaliser moyennant une hydrogénation catalytique, réduction avec du sodium dans l'ammoniaque liquide, hydrazinolyse, acidolyse comme par exemple en utilisant l'acide bromhydrique dans l'acide acétique, l'acide bromhydrique dans l'acide trifluoroacétique, l'acide chlorhydrique dans l'acide acétique ou en utilisant l'acide trifluoroacétique, ainsi que moyennant une hydrolyse avec un alcali, une hydrolyse catalysée par des enzymes ou en employant d'autres méthodes bien connues dans la synthèse des peptides.

La réaction s'effectue en l'absence d'autres solvants ou bien dans un solvant tel qu'un alcool comme par exemple l'éthanol, le méthanol ou l'isopropanol, le diméthylformamide, le diméthylsulfoxyde, l'acide acétique, l'acide trifluoroacétique ou l'eau.

Les températures les plus appropriées varient entre -15°C et celle de reflux du solvant, et le temps de réaction est compris entre dix minutes et 24 heures.

Méthode C

Par réaction d'un composé de formule générale VIII dans laquelle $R_5$, $R_6$, $R_7$, $R_6$ et n ont les significations mentionnées précédemment et dans laquelle $R_{11}$ est un radical diphénylméthyle, composés déjà décrits dans la demande de brevet européen 90401860.3, avec un composé de formule générale P1-A-P2, ou un composé de formule générale P1-A dans lequel P1, A et P2 ont les significations mentionnées précédemment et en utilisant les techniques de formation de liaisons peptidiques mentionnées dans la Méthode A, on obtient un composé de formule générale VII.

Par élimination des groupes protecteurs $R_{11}$ et P1, d'un composé de formule générale VII, moyennant des procédés déjà mentionnés dans le paragraphe Méthode B, on obtient des composés de formule générale VI.

Par réaction d'un composé de formule générale IV dans laquelle $R_1$, $R_2$, $R_3$, $R_4$, $R_9$, $R_{10}$ et X ont les significations mentionnées précédemment et Z représente un atome d'halogène, de préférence fluor ou chlore, avec une azétidine de formule générale VI, on obtient les composés de formule générale I. La réaction s'effectue en présence d'un solvant approprié, comme par exemple le diméthylformamide, le diméthylsulfoxyde, la pyridine, les trialkylamines telles que la triéthylamine, le dichlorométhane, le chloroforme, ainsi que des éthers tels que le tétrahydrofuranne ou le dioxanne, ou un mélange de ces solvants. Les températures les plus appropriées varient entre la température ambiante et la température de reflux du solvant, et le temps de réaction est compris entre 1 heure et 24 heures.

Méthode D

Par hydrogénolyse d'un composé de formule générale VII, dans laquelle $R_5$, $R_6$, $R_7$, $R_6$, A, P1, n et $R_{11}$ ont les significations mentionnées précédemment, moyennant l'utilisation d'un catalyseur de palladium, de préférence $Pd(OH)_2/C$ sous atmosphère d'hydrogène et dans un solvant approprié tel que l'alcool, comme par exemple l'éthanol ou le méthanol, et à une pression d'hydrogène comprise entre 1 atm. et 20 atm. et les températures appropriées varient entre 20°C et 70°C, on obtient un composé de formule générale V.

Par réaction d'un composé de formule générale IV, dans laquelle $R_1$, $R_2$, $R_3$, $R_4$, $R_9$, $R_{10}$ et X ont les significations mentionnées précédemment, et Z représente un atome d'halogène, de préférence fluor ou chlore, avec une azétidine de formule générale V, on obtient les composés de formule générale III. La réaction s'effectue en présence d'un solvant approprié, par exemple le diméthylformamide, le diméthylsulfoxyde, la pyridine, les trialkylamines telles que la triéthylamine, le dichlorométhane, le chloroforme ainsi que des éthers tels que le tétrahydrofuranne ou le dioxanne, ou un mélange de ces solvants. Les températures les plus appropriées varient entre la température ambiante et la température de reflux du solvant, et le temps de réaction est compris entre 1 heure et 24 heures.

Méthode E

Par réaction d'un composé de formule générale I avec un acide minéral ou organique dans un solvant approprié tel que l'éthanol ou le méthanol, on obtient le sel correspondant.

Dans les exemples suivants, on indiquera la préparation de nouveaux dérivés selon l'invention. On décrira également quelques formes d'emploi typiques pour les différents domaines d'application.

Les exemples ci-après, donnés à simple titre d'illustration, ne doivent cependant en aucune façon limiter l'étendue de l'invention.

Exemple 2aA. - Préparation de l'acide 7-[(2S,3R)-2-méthyl-3 -N-CBZ-Ala-amino-1-azétidinyl]-1-cyclopropyl-6-fluoro-1,4-dihydro-4-oxo-1,8-naphtyridine-3-carboxylique.

On ajoute à une solution d'acide 7-[(2S,3R)-2-méthyl-3-amino-1-azétidinyl]-1-cyclopropyl-6-fluoro-1,4-dihydro-4-oxo-1,8-naphtyridine-3-carboxylique (1a) (700 mg, 2,11 mmoles) et N-méthylmorpholine (213 mg, 2,11 mmoles) dans du diméthylformamide sec (30 ml) refroidie à 0°C, l'ester N-CBZ-Ala-N-hydroxysuccinimide (742 mg, 2,31 mmoles) et on maintient à cette température pendant 1 heure puis à température ambiante pendant 8 heures. On additionne la solution résultante sur une solution d'acide chlorhydrique (200 ml, 0,5N) et il se forme un précipité que l'on filtre, on lave avec de l'eau, on sèche avec $P_2O_5$ et on obtient l'acide 7-[(2S,3R)-2-méthyl-3-N-CBZ-Ala-amino-1-azétidinyl]-1-cyclopropyl-6-fluoro-1,4-dihydro-4-oxo-1,8-naphtyridine-3-car boxylique (2aA) (1,1 g, 2,04 mmoles) de point de fusion 211-3°C.

$[\alpha]_D^{20}$ = +27,7 (c = 0,78, DMSO)

Données spectroscopiques

RMN $^1H$ (100 MHz) ($DMSO-d_6$/TFA-d1) : 1,11 (m,4H) ; 1,21 (d,J = 7,0 Hz, 3H) ; 1,59 (d,J = 6,1 Hz,3H) ; 3,60 (m,1H) ; 3,85-4,70 (a.c., 5H) ; 5,01 (s,2H) ; 7,30 (s,5H) ; 7,97 (d,J = 11,5 Hz, 1H) ; 8,56 (s,1H).

IR (KBr) : 3325, 1720, 1680, 1632, 1509, 1449, 1328 $cm^{-1}$.

Exemple 3aA. - Préparation de l'acide 7-[(2S,3R)-2-méthyl-3-Ala-amino-1-azétidinyl]-1-cyclopropyl-6-fluoro-1,4-dihydro-4-oxo-1,8-naphtyridine-3-carboxylique.

On ajoute 80 mg de 10% -Pd/C à une solution d'acide 7-[(2S,3R)-2-méthyl-3-N-CBZ-Ala-amino-1-azétidinyl]-1-cyclopropyl-6-fluoro-1,4-dihydro-4-oxo-1,8-naphtyridine-3-carboxylique (2aA) (960 mg, 1,78 mmoles) dans 80 ml de diméthylformamide, et on maintient sous atmosphère d'hydrogène pendant 24 heures. On filtre le catalyseur et on lave bien avec du diméthylformamide. On fait évaporer le solvant à pression réduite, et le solide résultant se cristallise dans un mélange éthanol-eau, et l'on obtient l'acide 7-[(2S,3R)-2-méthyl-3-Ala-amino-1-azétidinyl]-1-cyclopropyl-6-fluoro-1,4-dihydro-4-oxo-1,8-naphtyridine-3-carboxylique (3aA) (500 mg, 1,23 mmoles).

Pf. = 220-222°C

$[\alpha]_D^{20}$ = +16,9 (c = 0,75, DMSO)

RMN $^1H$ (100 MHz) ($DMSO-d_6$/TFA) : 1,16 (m,4H), 1,39 (d,J=7,00 Hz, 3H), 1,65 (d,J = 6,2 Hz, 3H), 3,55-4,00 (m,2H), 4,00-4,80 (a.c., 4H), 8,02 (d,J = 11,6, 1H), 8,15 (é, 3H), 8,6 (s,1H), 8,95 (m,1H).

IR (KBr) : 3630-2420 (é), 1630, 1510, 1500, 1450, 1362, 1325 $cm^{-1}$.

Exemple 4aA. - Préparation du chlorhydrate de l'acide 7-[(2S,3R)-2-méthyl-3-Ala-amino-1-azétidinyl]-1-cyclopropyl-6-fluoro-1,4-dihydro-4-oxo-1,8-naphtyridine-3-carboxylique.

On traite 0,35 g (0,86 mmole) d'acide 7-[(2S,3R)-2-méthyl-3-Ala-amino-1-azétidinyl]-1-cyclopropyl-6-fluoro-1,4-dihydro-4-oxo-1,8-naphtyridine-3-carboxylique (3aA) avec une solution d'EtOH-Hcl, on fait évaporer le solvant à pression réduite et on obtient 0,37 g (0,84 mmole) du chlorhydrate de l'acide 7-[(2S,3R)-2-méthyl-3-Ala-amino-1-azétidinyl]-1-cyclopropyl-6-fluoro-1,4-dihydro-4-oxo-1,8-naphtyridine-3-carboxylique (4aA).

Pf. 190-192°C

$[\alpha]_D^{20}$ = +16,2 (c = 0,88, DMSO)

RMN $^1H$ (100 MHz) $DMSO-d_6$/TFA) : 1,10 (m,4H) 1,38 (d,J = 7,0 Hz, 3H), 1,63 (d,J = 6,2 Hz, 3H), 3,50-4,00 (m,2H), 4,00-4,80 (a.c., 4H), 8,00 (d,J = 11,5 Hz, 1H), 8,16 (é, 3H), 8,58 (s,1H), 9,13 (m,1H).

IR (KBr) 3620-2400 (é), 1718, 1686, 1631, 1561, 1490, 1449, 1328.

Exemple 2aL. - Préparation de l'acide 7-[(2S,3R)-2-méthyl-3-N-t-BOC-Leu-amino-1-azétidinyl]-1-cyclopropyl-6-fluoro-1,4-dihydro-4-oxo-1,8-naphtyridine-3-carboxylique.

A une solution de l'acide 7-[(2S,3R)-2-méthyl-3-amino-1-azétidyl]-1-cyclopropyl-6-fluoro-1,4-dihydro-4-oxo-1,8-naphtyridine-3-carboxylique (1a) (400 mg, 1,20 mmole) et de N-méthylmorpholine (121 mg, 1,20 mmole) dans le diméthylformamide sec (20 ml) et refroidi à 0°C, on ajoute du N-t-BOC-Leu N-hydroxysuccinimide ester (433 mg, 1,32 mmole) et on maintient cette température pendant une heure, et à température ambiante pendant une nuit. On verse la solution résultante sur une solution d'acide chlorhydrique (200 ml, 0,5N) et il apparaît un précipité que l'on filtre, qu'on lave à l'eau et que l'on sèche dans un dessicateur avec $P_2O_5$, et l'on obtient l'acide 7-[(2S,3R)-2-méthyl-3-N-t-BOC-Leu-amino-1-azétidinyl]-1-cyclopropyl-6-fluoro-1,4-dihydro-4-oxo-1,8-naphtyridine-3-carboxylique (2aL), (600 mg, 1,10 mmole).

Pf. 117-120°C

$[\alpha]_D^{20}$ = +17,0 (c = 0,71, DMSO).

RMN $^1H$ (100 MHz) ($DMSO-d_6$/$TFA-d_1$) : 0,85 (d,J = 5,9 Hz, 6H), 1,14 (m,4H), 1,35 (s,12H), 1,59 (d,J = 5,9 Hz, 3H), 3,55-4,70 (a.c. 6H), 7,97 (d,J = 11,5 Hz,1H), 8,57 (s,1H).

IR (KBr) : 3318, 2962, 1719, 1631, 1509, 1447, 1368, 1331 $cm^{-1}$.

Exemple 3aL. - Préparation de l'acide 7-[(2S,3R)-2-méthyl-3-Leu-amino-1-azétidinyl]-1-cyclopropyl-6-fluoro-1,4-dihydro-1,8-naphtyridine-3-carboxylique.

On mélange à froid 0,54 g (0,99 mmole) de l'acide 7-[(2S,3R)-2-méthyl-3-N-t-BOC-Leu-amino-1-azétidinyl]-1-cyclopropyl-6-fluoro-1,4-dihydro-4-oxo-1,8-naphtyridine-3-carboxylique (2aL) et 15 ml d'acide trifluoracétique et on maintient pendant une heure à la température ambiante. On y ajoute de l'éther et on filtre le précipité qui apparaît, et on le lave à l'éther. On dissout le sel ainsi formé dans l'eau et on ajuste à pH environ 7,6 avec $NH_3$, et il apparaît un précipité que l'on filtre avec l'eau, qu'on lave et qu'on sèche dans un dessicateur avec $P_2O_5$, et on obtient 0,25 g (0,56 mmole) d'acide 7-[(2S,3R)-2-méthyl-3-Leu-amino-1-azétidinyl]-1-cyclopropyl-6-fluoro-1,4-dihydro-4-oxo-1,8-naphtyridine-3-carboxylique (3aL).

Pf. = 216-218°C

$[\alpha]_D^{20}$ = +9,7 (c = 0,76, DMSO)

RMN $^1H$ (100 MHz) (DMSO-$d_6$/TFA) : 0,92 (d,J = 4,8 Hz, 6H, 1,13 (m,4H), 1,65 (d,J = 5,9 Hz, 6H), 3,68 (m,2H), 4,05-4,80 (m,4H), 8,03 (d,J = 11,7 Hz, 1H), 8,15 (é,3H), 8,60 (s,1H), 9,09 (m,1H).

IR (KBr) : 3331, 2962, 1724, 1636, 1571, 1509, 1449 $cm^{-1}$.

Exemple 4aL. - Préparation du chlorhydrate de l'acide 7-[(2S,3R)-2-méthyl-3-Leu-amino-1-azétidinyl]-1-cyclopropyl-6-fluoro-1,4-dihydro-4-oxo-1,8-naphtyridine-3-carboxylique.

On traite 0,20 g (0,45 mmole) d'acide 7-[(2S, 3R)-2-méthyl-3-Leu-amino-1-azétidinyl]-1-cyclopropyl-6-fluoro -1,4-dihydro-4-oxo-1,8-naphtyridine-3-carboxylique (3aL) avec une solution d'EtOH-ClH, on fait évaporer le solvant à pression réduite, et on obtient 0,21 g (0,44 mmole) de chlorhydrate de l'acide 7-[(2S,3R)-2-méthyl-3-Leu-amino-1-azétidinyl]-1-cyclopropyl-6-fluoro-1,4-dihydro -4-oxo-1,8-naphtyridine-3-carboxylique (4aL).

Pf. = 181-184°C

$[\alpha]_D^{20}$ = +23,8 (c = 0,75, DMSO)

RMN $^1H$ (100 MHz) (DMSO-$d_6$/TFA) : 0,89 (d,J = 5,2 Hz, 6H), 1,11 (m, 4H), 1,63 (d,J = 5,5 Hz, 6H), 3,43 (m,2H), 4,05-4,80 (a.c., 4H), 8,01 (d,J = 11,4 Hz, 1H), 8,15 (é, 3H), 8,58 (s,1H), 9,19 (m,1H).

IR (KBr) : 3600-2400 (é), 1718, 1687, 1630, 1562, 1512, 1449, 1325 $cm^{-1}$.

Exemple 2bA. - Préparation de l'acide 7-(3-N-CBZ-Ala-amino-1-azétidinyl)-1-(2,4-difluorophényl)-6-fluoro-1,4-dihydro-4-oxo-1,8-naphtyridine-3-carboxylique.

A une solution d'acide 7-(3-amino-1-azétidinyl)-1-(2,4-difluorophényl)-6-fluoro-1,4-dihydro-4-oxo-1,8-naphtyridine -3-carboxylique (1b) (780 mg, 2 mmoles) et de N-méthylmorpholine (202 mg, 2 mmole) dans le DMF sec (30 ml) et refroidie à 0°C, on ajoute le N-CBZ-Ala-N-hydroxysuccinimide ester (704 mg, 2,2 mmole) et on maintient à cette température pendant une heure, et à température ambiante pendant une nuit. On verse la solution résultante dans une solution d'HCl (200 ml, 0,5N) et il apparaît un précipité qu'on filtre et qu'on lave avec $H_2O$, et qu'on sèche sur $P_2O_5$. On obtient 1,06 g (1,78 mmole) de l'acide 7-(3-N-CBZ-Ala-amino-1-azétidinyl)-1-(2,4-difluorophényl)-6-fluoro-1,4-dihydro-4-oxo-1,8-naphtyridine -3-carboxylique (2bA).

Pf. = 205-208°C

$[\alpha]_D^{20}$ = +11,9 (c = 0,63, DMSO)

RMN $^1H$ (100 MHz) (DMSO-$d_6$/TFA-$d_1$) : 1,18 (d,J = 7,0 Hz, 3H), 3,70-4,70 (a.c., 6H), 4,98 (s,2H), 7,05-7,80 (a.c., 8H, ($\delta$ = 7,27,s)), 7,97 (d,J = 11,3Hz, 1H), 8,71 (s,1H).

IR (KBr) : 1449, 1509, 1631, 1656, 1719, 3318 $cm^{-1}$.

Exemple 3bA. - Préparation de l'acide 7-(3-Ala-amino-1-azétidinyl)-1-(2,4-difluorophényl)-6-fluoro-1,4-dihydro-4-oxo-1,8-naphtyridine-3-carboxylique.

On dissout 0,8 g (1,34 mmole) de l'acide 7-(3-N-CBZ-Ala-amino-1-azétidinyl)-1-(2,4-difluorophényl)-6-fluoro -1,4-dihydro-4-oxo-1,8-naphtyridine-3-carboxylique (2bA) dans 2 ml d'acide acétique. A cette solution, on ajoute 10 ml d'une solution d'acide acétique saturée en acide bromhydrique. On maintient à température ambiante pendant une heure, on ajoute de l'éther et il apparaît un précipité qu'on filtre et qu'on lave à l'éther. On dissout le sel dans l'eau et on ajuste à pH 7,6, et un solide se précipite, que l'on filtre, qu'on lave avec de l'eau et qu'on sèche dans un dessicateur avec $P_2O_5$, et on obtient 0,43 g (0,93 mmole) d'acide 7-(3-Ala-amino -1-azétidinyl)-1-(2,4-difluorophényl)-6-fluoro-1,4-dihydro-4-oxo-1,8-naphtyridine-3-carboxylique (3bA).

Pf. = 150-152°C

$[\alpha]_D^{20}$ = +7,80 (c = 0,59, DMSO)

RMN $^1H$ (100 MHz) ($d_6$-DMSO/TFA) (T = 310 K) : 1,33 (d,J = 7,0 Hz, 3H), 3,70-4,15 (m,3H), 4,15-4,70 (m,3H), 7,10-7,80 (a.c., 3H), 8,01 (d,J = 11,2 Hz, 1H), 8,1 (é, 3H), 8,72 (s,1H), 9,10 (m,1H)

IR (KBr) : 3418, 3262, 3075, 1636, 1561, 1510, 1459 $cm^{-1}$.

Exemple 4bA. - Préparation du chlorhydrate de l'acide 7-(3-Ala-amino-1-azétidinyl)-1-(2,4-difluorophényl)-6-fluoro-1,4-dihydro-4-oxo-1,8-naphtyridine-3-carboxylique.

On traite 0,33 g (0,71 mmole) d'acide 7-(3-Ala-amino-1-azétidinyl)-1-(2,4-difluorophényl)-6-fluoro-1,4-dihydro-4-oxo-1,8-naphtyridine-3-carboxylique (3bA) avec une solution d'EtOH-ClH, on fait évaporer le solvant à pression réduite, et on obtient 0,35 g (0,70 mmole) de chlorhydrate de l'acide 7-(3-Ala-amino-1-azétidinyl)-1(2,4-difluorophényl)-6-fluoro-1,4-dihydro-4-oxo-1,8-naphtyridine -3-carboxylique (4bA).

Pf. = 204-207°C

$[\alpha]_D^{20}$ = +5,1 (c = 0,84, DMSO).

RMN $^1$H (100 MHz) (DMSO-$d_6$/TFA) : 1,31 (d,J = 6,5 Hz, 3H), 3,70-4,70 (m,6H), 7,10-7,80 (a.c. 3H), 7,85-8,15 (m,4H), 8,71 (s,1H), 8,94 (m,1H).

IR (KBr) : 3600-2700 (é), 1725, 1687, 1632, 1492, 1450.

Exemple 2bL. - Préparation de l'acide 7-(3-N-t-BOC-Leu-amino-1-azétidinyl)-1-(2,4-difluorophényl)-6-fluoro-1,4-dihydro-4-oxo-1,8-naphtyridine-3-carboxylique.

A une solution de l'acide 7-(3-amino-1-azétidinyl)-1-(2,4-difluorophényl)-6-fluoro-1,4-dihydro-4-oxo-1,8-naphtyridine-3-carboxylique (1b) (780 mg, 2 mmole) et de N-méthylmorpholine (202 mg, 2 mmole) dans le DMF sec (30 ml) et refroidie à 0°C, on ajoute du N-t-BOC-Leu-N-hydroxysuccinimide ester (721 mg, 2,2 mmole) et on maintient à cette température pendant une heure et à température ambiante pendant une nuit. On ajoute la solution résultante à une solution d'HCl (200 ml, 0,5N) et il apparaît un précipité qu'on filtre et qu'on lave avec de l'eau et qu'on sèche dans un dessicateur avec $P_2O_5$. On obtient 1,11 g (1,82 mmole) de l'acide 7-(3-N-t-BOC-Leu-amino-1-azétidinyl)-1-(2,4-difluorophényl)-6-fluoro-1,4-dihydro-4-oxo-1,8-naphtyridine-3-carb oxylique (2bL).

Pf. = 123-127°C

$[\alpha]_D^{20}$ = +8,2 (c = 0,75, DMSO),

RMN $^1$H (100 MHz) (DMSO-$d_6$/TFA-$d_1$) : 0,87 (d,J = 5,6 Hz, 6H), 1,38 (s,12H), 3,80-4,70 (m,6H), 7,15-7,90 (m,3H), 8,05 (d,J = 11,3, Hz, 1H), 8,78 (s,1H).

IR (KBr) : 3331, 2956, 1719, 1633, 1509, 1448 $cm^{-1}$.

Exemple 3bL. - Préparation de l'acide 7-(3-Leu-amino-1-azétidinyl)-1-(2,4-difluorophényl)-6-fluoro-1,4-dihydro-4-oxo-1,8-naphtyridine-3-carboxylique.

On mélange à froid 0,8 g (1,32 mmole) de l'acide 7-(3-N-t-BOC-Leu-amino-1-azétidinyl)-1-(2,4-difluorophényl)-6-fluoro-1,4-dihydro-4-oxo-1,8-naphtyridine-3-carboxylique (2bL) et 10 ml d'acide trifluoracétique et on maintient une heure à la température ambiante. On y ajoute de l'éther et on filtre le précipité qui apparaît, et on le lave à l'éther. On dissout le sel ainsi formé dans l'eau et on l'agite à pH 7,6 avec $NH_3$, et il apparaît un précipité qu'on filtre, qu'on lave à l'eau et qu'on sèche dans un dessicateur avec $P_2O_5$, et on obtient 0,56 g (1,11 mmole) de l'acide 7-(3-Leu-amino-1-azétidinyl)-1-(2,4-difluorophényl)-6-fluoro-1,4-dihydro-4-oxo-1,8-naphtyridine-3-carboxylique (3bL).

Pf. = 214-216°C

$[\alpha]_D^{20}$ = +3,8 (c = 0,55, DMSO).

RMN $^1$H (100 MHz) (DMSO-$d_6$/TFA) (T= 320 K) : 0,89 (d,J = 5,3 Hz, 6H), 1,57 (m,3H), 3,67 (m,1H), 4,02 (m,2H), 4,42 (m,3H), 7,19-7,86 (m,3H), 8,03 (d,J = 11,2 Hz, 1H), 8,16 (é, 3H), 8,74 (s,1H), 8,99 (m,1H).

IR (KBr) : 3600-2400 (é), 1725, 1637, 1561, 1509, 1460, 1357 $cm^{-1}$.

Exemple 4bL. - Préparation du chlorhydrate de l'acide 7-(3-Leu-amino-1-azétidinyl)-1-(2,4-difluorophényl)-6-fluoro-1,4-dihydro-4-oxo-1,8-naphtyridine-3-carboxylique.

On traite 0,45 g (0,89 mmole) d'acide 7-(3-Leu-amino-1-azétidinyl)-1-(2,4-difluorophényl)-6-fluoro-1,4-dihydro-4-oxo-1,8-naphtyridine-3-carboxylique (3bL) avec une solution d'EtOH-ClH, on fait évaporer le solvant à pression réduite, et l'on obtient un solide qu'on cristallise avec EtOH-éther, et l'on obtient 0,43 g (0,79 mmole) du chlorhydrate de l'acide 7-(3-Leu-amino-1-azétidinyl)-1-(2,4-difluorophényl)-6-fluoro-1,4-dihydro-4-oxo-1,8-naphtyridine-3-carboxylique (4bL).

Pf. = 190-195°C

$[\alpha]_D^{20}$ = +15,6 (c = 0,76, DMSO).

RMN $^1$H (100 MHz) (DMSO-$d_6$/TFA) : 0,87 (d,J = 5,1 Hz, 6H), 1,54 (m,3H), 3,60-4,70 (a.c., 6H), 7,15-7,80 (m,3H), 8,03 (d,J = 11,2 Hz, 1H), 8,16 (é, 3H), 8,75 (s,1H), 9,35 (m,1H).
IR (KBr) : 3600-2400 (é), 1718, 1687, 1631, 1512, 1450 $cm^{-1}$.

Exemple 2ca. - Préparation de l'acide 7-(3-méthyl-3-N-CBZ-D-Ala-amino-1-azétidinyl)-1-cyclopropyl-6,8-difluoro-1,4-dihydro-4-oxo-3-quinoléinecarboxylique.

On neutralise une solution de N-CBZ-D-Alanine (319 mg, 1,43 mmoles) dans 7 ml de tétrahydrofuranne sec et refroidie à -15°C avec de la N-méthylmorpholine (145 mg, 1,43 mmoles) et on additionne du chloroformiate d'isobutyle (195 mg, 1,43 mmoles). Cinq minutes plus tard, on additionne une suspension d'acide 7-(3-méthyl-3-amino-1-azétidinyl)-1-cyclopropyl-6,8-difluoro-1,4-dihydro-4-oxo-3-quinoléinecarboxylique (1c) (500 mg, 1,43 mmoles) dans 15 ml de diméthylformamide, on maintient le mélange à -15°C pendant 15 minutes et on laisse à température ambiante et sous forte agitation pendant 8 heures. On ajoute ensuite le mélange sur une solution froide de HCl (100 ml, 0,5N) et il se forme un précipité que l'on filtre, on lave avec de l'eau, on sèche avec $P_2O_5$ et on obtient 570 mg (1,03 mmoles) de l'acide 7-(3-méthyl-3-N-CBZ-D-Ala-amino-1-azétidinyl)-1-cyclopropyl-6,8-difluoro-1,4-dihydro-4-oxo-3-quinoléinecarboxylique (2ca), de point de fusion 138-141°C.

Données spectroscopiques :

RMN $^1H$ (100 MHz) (DMSO-$d_6$/TFA-$d_1$) : 0,85-1,28 (a.c., 7H), 1,54 (s,3H), 3,79-4,60 (a.c.,6H), 5,00 (s,2H), 7,30 (s,5H), 7,64 (d,J = 13,1Hz,1H), 8,55 (s,1H).
IR (KBr) : 3320, 1725, 1680, 1627, 1528, 1459, 1413, 1327 $cm^{-1}$.

Exemple 3ca. - Préparation de l'acide 7-(3-méthyl-3-D-Ala-amino-1-azétidinyl)-1-cyclopropyl-6,8-difluoro-1,4-dihydro-4-oxo-3-quinoléinecarboxylique.

A une solution de l'acide 7-(3-méthyl-3-N-CBZ-D-Ala-amino-1-azétidinyl)-1-cyclopropyl-6,8-difluoro-1,4-dihydro-4-oxoquinoléinecarboxylique (2ca) (196 mg, 0,35 mmole) dans 15 ml de diméthylformamide, on ajoute 15 mg de Pd /C à 10 % maintient sous atmosphère d'hydrogène pendant 24 heures. On filtre le catalyseur et on lave bien avec du diméthylformamide. On fait évaporer le solvant à pression réduite et le solide résultant cristallise dans un mélange d'éthanol-eau, et l'on obtient l'acide 7-(3-méthyl-3-D-Ala-amino-1-azétidinyl)-1-cyclopropyl-6,8-difluoro-1,4-dihydro-4-oxo-quinoléine-carboxylique (3ca) (63 mg, 0,15 mmole).
Pf. = 154-160°C
RMN $^1H$ (100 MHz) (DMSO-$d_6$/TFA) : 1,15 (m,4H), 1,36 (d,J = 6,5 Hz, 3H), 1,57 (s,3H), 3,65-4,70 (a.c., 6H), 7,67 (d,J = 11,4 Hz, 1H), 8,05 (é, 3H), 8,56 (s,1H), 8,90 (m,1H).
IR (KBr) : 3600-2400 (é), 1718, 1675, 1628, 1528, 1467, 1326 $cm^{-1}$.

Exemple 4ca. - Préparation du chlorhydrate de l'acide 7-(3-méthyl-3-D-Ala-amino-1-azétidinyl)-1-cyclopropyl-6,8-difluoro-1,4-dihydro-4-oxo-3-quinoléinecarboxylique.

On traite 44 mg (0,11 mmole) d'acide 7-(3-méthyl-3-D-Ala-amino-1-azétidinyl)-1-cyclopropyl-6,8-difluoro-1,4-dihydro-4-oxo-quinoléinecarboxylique (3ca) avec une solution d'EtOH-HCl, on fait évaporer le solvant à pression réduite et l'on obtient 42 mg (0,09 mmole) du chlorhydrate de l'acide 7-(3-méthyl-3-D-Ala-amino-1-azétidinyl)-1-cyclopropyl-6,8-difluoro-1,4-dihydro-4-oxo-3-quinoléinecarboxylique (4ca).
Pf. = 181-185°C
RMN $^1H$ (100 MHz) (DMSO-$d_6$/TFA) : 1,12 (m,4H), 1,34 (d,J = 5,5 Hz, 3H), 1,55 (s,3H), 3,70-4,70 (a.c., 6H), 7,68 (d,J = 12,7 Hz, 1H), 8,01 (é, 3H), 8,55 (s,1H), 8,79 (m,1H).
IR (KBr) : 3600-2400 (é), 1718, 1688, 1627, 1528, 1465, 1328 $cm^{-1}$.

Exemple 3da. - Préparation de l'acide 7-(3-méthyl-3-D-Ala-amino-1-azétidinyl)-1-cyclopropyl-6-fluoro-8-chloro-1,4-dihydro-4-oxo-3-quinoléinecarboxylique.

On neutralise une solution de N-CBZ-D-alanine (135 g, 6 mmoles) dans 30 ml de tétrahydrofuranne sec et refroidi à -150°C avec de la N-méthylmorpholine (0,61 g, 6 mmoles) et on ajoute du chloroformiate d'isobutyle (0,82 g, 6 mmoles) 5 minutes plus tard, on ajoute une solution de 3-amino-3-méthyl-1-benzhydrylazétidine (1,52 g, 6 mmoles) dans 20 ml de tétrahydrofuranne, on maintient le mélange à -10°C pendant 15 minutes. On laisse arriver la solution à la température ambiante, on filtre le précipité qui se forme et on fait évaporer le filtrat jusqu'à siccité, et l'on obtient, une fois cristallisé dans AcOEt-hexane, la 3-méthyl-3-N-CBZ-D-Ala-amino-1-benzhydrylazétidine, (1,83 g, 4 mmole) de point de fusion 90-98°C.

On traite 1,68 g (3,6 mmole) de 3-méthyl-3-N-CBZ-D-Ala-amino-1-benzhydrylazétidine avec de l'éthanol saturé en acide chlorhydrique, on fait évaporer le solvant à pression réduite, et l'on obtient 1,69 g (3,4 mmole) de chlorhydrate de 3-méthyl-3-N-CBZ-D-Ala-amino-1-benzhydrylazétidine, de point de fusion 93-97°C.

A une solution de chlorhydrate de 3-méthyl-3-N-CBZ-D-Ala-amino-1-benzhydrylazétidine (1,63 g, 3,3 mmoles) dans 150 ml de méthanol, on ajoute 160 mg de Pd $(OH)_2/C$ (20%) et on laisse sous atmosphère d'hydrogène (16 atm.) pendant une nuit. On filtre le catalyseur, on fait évaporer le solvant à pression réduite, et l'on obtient le chlorhydrate de 3-méthyl-3-D-Ala-aminoazétidine (600 mg, 3,1 mmole).

On mélange (100 mg, 0,33 mmole) de l'acide 1-cyclopropyl-6,7-difluoro-8-chloro-1,4-dihydro-4-oxo-3-quinoléinecarboxylique, (129 mg, 0,66 mmole) de chlorhydrate de 3-méthyl-3-D-Ala-aminoazétidine et (290 mg, 2,8 mmole) de triéthylamine dans 8 ml de pyridine anhydre. On maintient la solution à 70°C pendant 15 heures. On fait évaporer le solvant à pression réduite, et le produit brut résultant se cristallise à partir d'un mélange d'éthanol-eau, et l'on obtient 66 mg (0,15 mmole) d'acide 7-(3-méthyl-3-D-Ala-amino-1-azétidinyl)-1-cyclopropyl-6-fluoro-8-chloro-1,4-dihydro-4-oxo-3-quinoléinecarboxylique (3da).

Pf. = 167-171°C

RMN $^1H$ (100 MHz) ($DMSO-d_6/TFA$) : 0,90-1,20 (m,4H), 1,37 (d,J = 7,0 Hz, 3H), 1,57 (s,3H), 3,81 (m,1H), 4,10-4,70 (a.c., 5H), 7,75 (d,J = 13,7 Hz, 1H), 8,1 (m, 3H), 8,72 (s,1H), 9,08 (m,1H).

IR (KBr) : 3600-2400 (é), 1718, 1668, 1618, 1543, 1443, 1405, 1312 $cm^{-1}$.

Exemple 4da. - Préparation du chlorhydrate de l'acide 7-(3-méthyl-3-D-Ala-amino-1-azétidinyl)-1-cyclopropyl-6 -fluoro-8-chloro-1,4-dihydro-4-oxo-3-quinoléinecarboxylique.

On traite 35 mg (0,08 mmole) d'acide 7-(3-méthyl -3-D-Ala-amino-1-azétidinyl)-1-cyclopropyl-6-fluoro-8-chloro-1,4-dihydro-4-oxo-3-quinoléinecarboxylique (3da) avec une solution d'EtOH-HCl, on fait évaporer le solvant à pression réduite et l'on obtient 36 mg (0,07 mmole) du chlorhydrate de l'acide 7-(3-méthyl -3-D-Ala-amino-1-azétidinyl)-1-cyclopropyl-6-fluoro-8-chloro-1,4-dihydro-4-oxo-3-quinoléinecarboxylique.

Pf. = 176-180°C.

RMN $^1H$ (100 MHz) ($DMSO-d_6/TFA$) : 0,90-1,20 (m,4H), 1,36 (d,J = 6,9 Hz, 3H), 1,56 (s,3H), 3,80 (m,1H), 4,10-4,70 (a.c., 5H), 7,75 (d,J = 13,3 Hz, 1H), 8,00 (m,3H), 8,70 (s,1H), 9,00 (m,1H).

IR (KBr) : 3600-2400 (é), 1718, 1681, 1625, 1450, 1406, 1318 $cm^{-1}$.

**TABLEAU I**

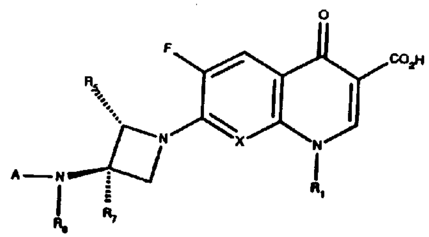

| Exemple | $R_1$ | $R_5$ | $R_7$ | $R_8$ | X | A | stereochimie | Sel | p.f. (°C) | $[\alpha]_D^{20}$ (DMSO) | IR ($cm^{-1}$) (KBr) |
|---|---|---|---|---|---|---|---|---|---|---|---|
| 1a | 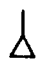 | $CH_3$ | H | H | N | H | (2S, 3R) | - | | - | |
| 1b | 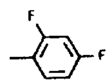 | H | H | H | N | H | - | - | | - | |
| 1c | 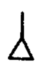 | H | $CH_3$ | H | C-F | H | - | - | | - | |
| 1d | 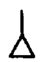 | H | $CH_3$ | H | C-Cl | H | - | - | | - | |
| 1e | 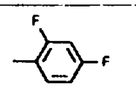 | $CH_3$ | H | H | C-H | H | (2S, 3R) | - | | - | |
| 1f | 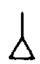 | $CH_3$ | H | H | N | H | (2R, 3S) | - | | - | |
| 2aA | 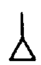 | $CH_3$ | H | H | N | N-CBZ-Ala | (2S, 3R) | - | 211-213 | +27,7 (c=0,78) | 3325, 1720, 1680, 1632, 1509, 1449, 1328 |

**TABLEAU I (Cont.)**

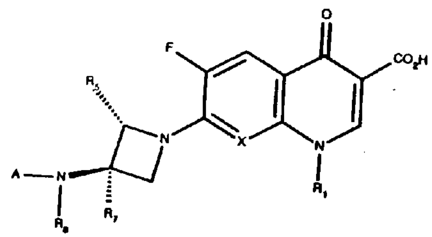

| Exemple | $R_1$ | $R_5$ | $R_7$ | $R_8$ | X | A | stereochimie | Sel | p.f. (°C) | $[\alpha]_D^{20}$ (DMSO) | IR ($cm^{-1}$) (KBr) |
|---|---|---|---|---|---|---|---|---|---|---|---|
| 3aA | 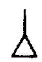 | $CH_3$ | H | H | N | Ala | (2S,3R) | - | 220-222 | +16,9 (c=0,75) | 3630-2420 (é), 1630, 1510, 1500, 1450, 1362, 1325 |
| 4aA | 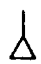 | $CH_3$ | H | H | N | Ala | (2S,3R) | HCl | 190-192 | +16,2 (c=0,88) | 3620-2400 (é), 1718, 1686, 1631, 1561, 1490, 1449, 1328 |
| 2aL | 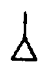 | $CH_3$ | H | H | N | N-t-BOC-Leu | (2S,3R) | - | 117-120 | +17,0 (c=0,71) | 3318, 2962, 1719, 1631, 1509, 1447, 1368, 1331 |
| 3aL | 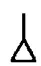 | $CH_3$ | H | H | N | Leu | (2S,3R) | - | 216-218 | +9,7 (c=0,76) | 3331, 2962, 1724, 1636, 1571, 1509, 1449 |
| 4aL | 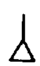 | $CH_3$ | H | H | N | Leu | (2S,3R) | HCl | 181-184 | +23,8 (c=0,75) | 3600-2400 (é), 1718, 1687, 1630, 1562, 1512, 1449, 1325 |
| 2bA | 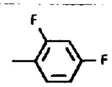 | H | H | H | N | N-CBZ-Ala | - | - | 205-208 | +11,9 (c=0,63) | 3318, 1719, 1656, 1631, 1509, 1449 |
| 3bA | 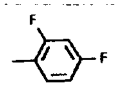 | H | H | H | N | Ala | - | - | 150-152 | +7,8 (c=0,59) | 3418, 3262, 3075, 1636, 1561, 1510, 1459 |

**TABLEAU I (Cont.)**

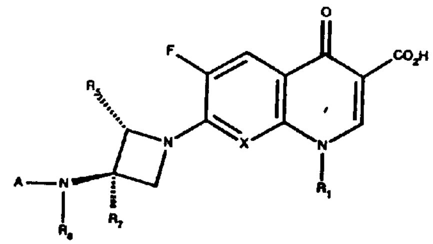

| Exemple | $R_1$ | $R_5$ | $R_7$ | $R_8$ | X | A | stereochimie | Sel | p.f. (°C) | $[\alpha]_D^{20}$ (DMSO) | IR ($cm^{-1}$) (KBr) |
|---|---|---|---|---|---|---|---|---|---|---|---|
| 4bA | 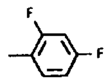 | H | H | H | N | Ala | - | HCl | 204-207 | +5,1 (c=0,84) | 3600-2700 (é), 1725, 1687, 1632, 1492, 1450 |
| 2bL | 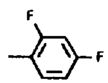 | H | H | H | N | N-t-BOC-Leu | - | - | 123-127 | +8,2 (c=0,75) | 3331, 2956, 1719, 1633, 1509, 1448 |
| 3bL | 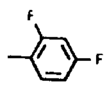 | H | H | H | N | Leu | - | - | 214-216 | +3,8 (c=0,55) | 3600-2400 (é), 1725, 1637, 1561, 1509, 1460, 1357 |
| 4bL | 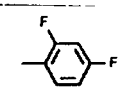 | H | H | H | N | Leu | - | HCl | 190-195 | +15,6 (c=0,76) | 3600-2400 (é), 1718, 1687, 1631, 1512, 1450 |
| 2bG | 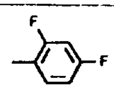 | H | H | H | N | N-t-BOC-Gly | - | - | 228-230 | - | 3331, 1720, 1659, 1632, 1509, 1445, 1151 |
| 3bG | 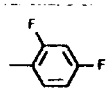 | H | H | H | N | Gly | - | - | 235-236 (dec.) | - | 3600-2400 (é), 1638, 1568, 1512, 1466, 1370 |
| 4bG | 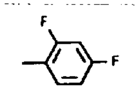 | H | H | H | N | Gly | - | HCl | 274-277 (dec.) | - | 3600-2400 (é), 1710, 1671, 1632, 1612, 1571, 1509, 1460 |

**TABLEAU I (Cont.)**

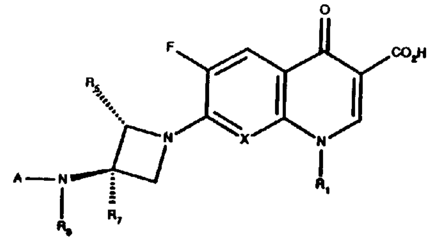

| Exemple | $R_1$ | $R_5$ | $R_7$ | $R_8$ | X | A | stereochimie | Sel | p.f. (°C) | $[\alpha]_D^{20}$ (DMSO) | IR ($cm^{-1}$) (KBr) |
|---|---|---|---|---|---|---|---|---|---|---|---|
| 2ca | 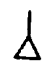 | H | $CH_3$ | H | C-F | N-CBZ-D-Ala | - | - | 138-141 | - | 3320, 1725, 1680, 1627, 1528, 1459, 1413, 1327 |
| 3ca | 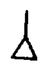 | H | $CH_3$ | H | C-F | D-Ala | - | - | 154-160 | - | 3620-2400 (é), 1718, 1675, 1628, 1528, 1467, 1326 |
| 4ca | 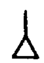 | H | $CH_3$ | H | C-F | D-Ala | - | HCl | 181-185 | - | 3600-2400 (é), 1718, 1688, 1627, 1528, 1465, 1328 |
| 2da | 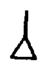 | H | $CH_3$ | H | C-Cl | N-CBZ-D-Ala | - | - | 144-149 | - | 3325, 1725, 1622, 1438 |
| 3da | 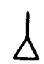 | H | $CH_3$ | H | C-Cl | D-Ala | - | - | 167-171 | - | 3600-2400 (é), 1718, 1668, 1618, 1543, 1443, 1405, 1312 |
| 4da | 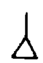 | H | $CH_3$ | H | C-Cl | D-Ala | - | HCl | 176-180 | - | 3600-2400 (é), 1718, 1681, 1625, 1450, 1406, 1318 |
| 2ea | 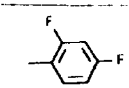 | $CH_3$ | H | H | C-H | N-CBZ-D-Ala | (2S, 3R) | - | 121-124 | -59,5 (c=0,63) | 3560-3200 (é), 1735, 1655, 1508, 1460, 1401, 1325 |

**TABLEAU I (Cont.)**

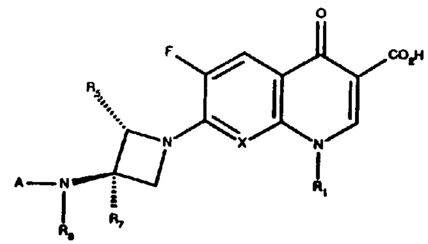

| Exemple | $R_1$ | $R_5$ | $R_7$ | $R_8$ | X | A | stereochimie | Sel | p.f. (°C) | $[\alpha]_D^{20}$ (DMSO) | IR ($cm^{-1}$) (KBr) |
|---|---|---|---|---|---|---|---|---|---|---|---|
| 3ea | 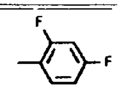 | $CH_3$ | H | H | C-H | D-Ala | (2S,3R) | - | 144-146 | -57,9 (c=0,43) | 3600-2500 (é), 1630, 1509, 1475, 1420, 1340 |
| 4ea | 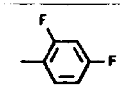 | $CH_3$ | H | H | C-H | D-Ala | (2S,3R) | HCl | 207-211 | - | 3600-2400 (é), 1718, 1687, 1637, 1506, 1462, 1393, 1318 |
| 2bN | 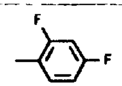 | H | H | H | N | N-t-BOC-Nva | - | - | 141-143 | +9,9 (c=0,61) | 3331, 1719, 1633, 1509, 1449 |
| 3bN | 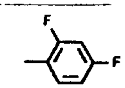 | H | H | H | N | Nva | - | - | 176-179 | +7,3 (c=0,54) | 3400-2500 (é), 1725, 1635, 1569, 1510, 1459, 1368, 1275 |
| 4bN | 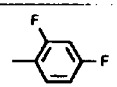 | H | H | H | N | Nva | - | HCl | 196-200 | +12,1 (c=077) | 3400-2500 (é), 1725, 1687, 1639, 1509, 1449 |
| 2aa | 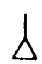 | $CH_3$ | H | H | N | N-CBZ-D-Ala | (2S,3R) | - | 177-182 | -20,8 (c=0,61) | 3368, 1720, 1632, 1509, 1448, 1331 |
| 3aa | 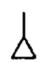 | $CH_3$ | H | H | N | D-Ala | (2S-3R) | - | 234-236 | +6,7 (c=0,79) | 3600-2500 (é), 1725, 1656, 1631, 1550, 1512, 1450, 1331 |

**TABLEAU I** (Cont.)

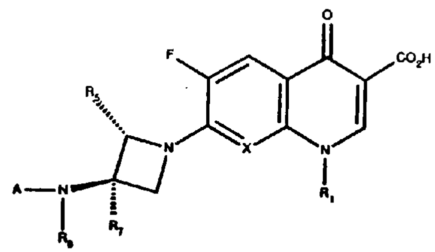

| Exemple | $R_1$ | $R_5$ | $R_7$ | $R_8$ | X | A | stereochimie | Sel | p.f. (°C) | $[\alpha]_D^{20}$ (DMSO) | IR ($cm^{-1}$) (KBr) |
|---|---|---|---|---|---|---|---|---|---|---|---|
| 4aa | 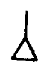 | $CH_3$ | H | H | N | D-Ala | (2S-3R) | HCl | 238-240 | +3,4 (c=0,73) | 3600-2400 (é), 1718, 1668, 1543, 1443, 1331 |
| 2ba | 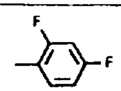 | H | H | H | N | N-CBZ-D-Ala | - | - | 200-203 | -12,6 (c=0,81) | 3293, 1719, 1656, 1631, 1543, 1509, 1449, 1331 |
| 3ba | 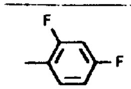 | H | H | H | N | D-Ala | - | - | 155-157 | -8,2 (c=0,69) | 3418, 3263, 3075, 1635, 1560, 1509, 1460 |
| 4ba | 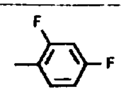 | H | H | H | N | D-Ala | - | HCl | 202-205 | -5,9 (c=0,53) | 3600-2400 (é), 1725, 1685, 1631, 1492, 1449 |
| 2fa | 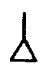 | $CH_3$ | H | H | N | N-CBZ-D-Ala | (2R,3S) | - | 207-210 | -27,1 (c=0,82) | 3312, 1718, 1681, 1632, 1543, 1512, 1325, 1237 |
| 3fa | 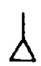 | $CH_3$ | H | H | N | D-Ala | (2R,3S) | - | 218-221 | -16,2 (c=0,64) | 3360-2400 (é), 1632, 1509, 1500, 1451, 1362, 1325 |
| 4fA | 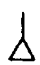 | $CH_3$ | H | H | N | D-Ala | (2R-3S) | HCl | 193-195 | -16,1 (c=0,67) | 3600-2400 (é), 1718, 1685, 1631, 1560, 1490, 1450, 1325 |

TABLEAU I (Cont.)

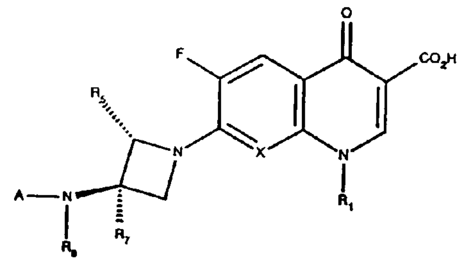

| Exemple | $R_1$ | $R_5$ | $R_7$ | $R_8$ | X | A | stereochimie | Sel | p.f. (°C) | $[\alpha]_D^{20}$ (DMSO) | IR ($cm^{-1}$) (KBr) |
|---|---|---|---|---|---|---|---|---|---|---|---|
| 2aAA | 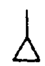 | $CH_3$ | H | H | N | N-CBZ-Ala-Ala | (2<u>S</u>-3<u>R</u>) | - | 121-123 | +14,8 (c=0,77) | 3300, 1718, 1680, 1630, 1447 |
| 3aAA | 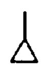 | $CH_3$ | H | H | N | Ala-Ala | (2<u>S</u>, 3<u>R</u>) | - | 194-198 | +12,0 (c=0,51) | 3600-2400, 1632, 1543, 1452, 1368, 1325 |
| 4aAA | 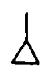 | $CH_3$ | H | H | N | Ala-Ala | (2<u>S</u>, 3<u>R</u>) | HCl | 188-191 | +16,6 (c=0,7) | 3600-2400, 1687, 1668, 1632, 1568, 1475, 1325 |
| 2eA | 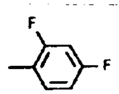 | $CH_3$ | H | H | C-H | N-CBZ-Ala | (2<u>S</u>, 3<u>R</u>) | - | 119-122 | -21,8 (c=0,7) | 3325, 1718, 1629, 1506, 1462, 1325 |
| 3eA | 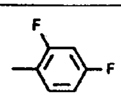 | $CH_3$ | H | H | C-H | Ala | (2<u>S</u>, 3<u>R</u>) | - | 152-156 | -40.5 (c=0,77) | 3600-2300, 1630, 1509, 1475, 1387, 1318 |
| 4eA | 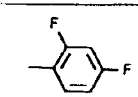 | $CH_3$ | H | H | C-H | Ala | (2<u>S</u>, 3<u>R</u>) | TsOH | 172-175 | -21,0 (c=0,9) | 3600-2300, 1718, 1687, 1630, 1520, 1466 |

**TABLEAU I** (Cont.)

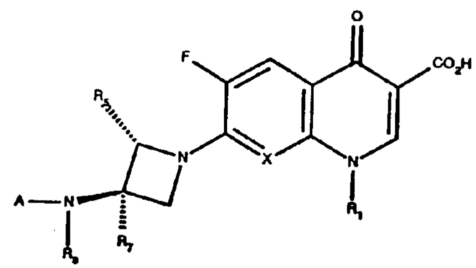

| Exemple | $R_1$ | $R_5$ | $R_7$ | $R_8$ | X | A | stereochimie | Sel | p.f. (°C) | $[\alpha]_D^{20}$ (DMSO) | IR ($cm^{-1}$) (KBr) |
|---|---|---|---|---|---|---|---|---|---|---|---|
| 2eN | 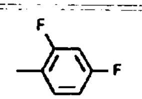 | $CH_3$ | H | H | C-H | N-BOC-Nval | (2S-3R) | - | 123-127 | -26,5 (c=0,84) | 3362, 1718, 1629, 1507, 1466, 1318 |
| 3eN | 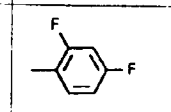 | $CH_3$ | H | H | C-H | Nval | (2S, 3R) | - | 205-209 | -17,0 (c=0,96) | 3600-2300, 1675, 1630, 1508, 1468, 1325 |
| 4eN | 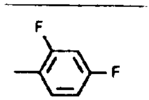 | $CH_3$ | H | H | C-H | Nval | (2S, 3R) | TsOH | 164-167 | -12,3 (c=0,79) | 3600-2300, 1718, 1681, 1629, 1507, 1466, 1325 |

**TABLEAU II**

| Exemple | Solvant | RMN-$^1$H (100 MH$_z$) |
|---|---|---|
| 2aA | DMSO-$d_6$/TFA-$d_1$ | 1,11 (m,4H); 1,21 (d,J = 7,0 Hz, 3H); 1,59 (d,J = 6,1 Hz, 3H); 3,60 (m,1H); 3,85-4,70 (a.c., 5H); 5,01 (s,2H); 7,30 (s,5H); 7,97 (d,J = 11,5 Hz, 1H); 8,56 (s, 1H) |
| 3aA | DMSO-$d_6$/TFA | 1,16 (m,4H); 1,39 (d,J = 7,00 Hz, 3H); 1,65 (d,J = 6,2 Hz, 3H); 3,55-4,00 (m,2H), 4,00-4,80 (a.c., 4H), 8,02 (d,J = 11,6 Hz, 1H), 8,15 é, 3H); 8,60 (s,1H); 8,95 (m,1H) |
| 4aA | DMSO-$d_6$/TFA | 1,10 (m,4H), 1,38 (d,J = 7,00 Hz, 3H); 1,63 (d,J = 6,2 Hz, 3H); 3,50-4,00 (m,2H); 4,00-4,80 (a.c., 4H); 8,00 (d,J = 11,5 Hz, 1H); 8,16 (é, 3H); 8,58 (s,1H); 9,13 (m,1H) |
| 2aL | DMSO-$d_6$/TFA-$d_1$ | 0,85 (d,J = 5,9 Hz, 6H); 1,14 (m,4H); 1,35 (s,12 H); 1,59 (d,J = 5,9 Hz, 3H); 3,55-4,70 (a.c., 6H); 7,97 (d,J = 11,5 Hz, 1H); 8,57 (s,1H) |
| 3aL | DMSO-$d_6$/TFA | 0,92 (d,J = 4,8 Hz, 6H); 1,13 (m,4H); 1,65 (d,J = 5,9 Hz, 6H); 3,68 (m,2H); 4,05-4,80 (m,4H); 8,03 (d,J = 11,7 Hz, 1H); 8,15 (é, 3H); 8,60 (s,1H); 9,09 (m,1H) |

TABLEAU II (Cont.)

| Exemple | Solvant | RMN-$^1$H (100 MH$_2$) |
|---|---|---|
| 4aL | DMSO-$d_6$/TFA | 0,89 (d,J = 5,2 Hz, 6H); 1,11 (m,4H); 1,63 (d,J = 5,5 Hz, 6H); 3,43 (m,2H); 4,05-4,80 (a.c., 4H); 8,01 (d,J = 11,4 Hz, 1H); 8,15 (é, 3H); 8,58 (s,1H); 9,19 (m,1H) |
| 2bA | DMSO-$d_6$/TFA-$d_1$ | 1,18 (d,J = 7,0 Hz, 3H); 3,70-4,70 (a.c., 6H); 4,98 (s,2H); 7,05-7,80 (a.c., 8H (δ = 7,27,s)); 7,97 (d,J = 11,3 Hz, 1H); 8,71 (s,1H) |
| 3bA | DMSO-$d_6$/TFA | 1.33 (d,J =7,0 Hz, 3H); 3,70-4,15 (m,3H); 4,15-4,70 (m,3H); 7,10-7,80 (a.c., 3H); 8,01 (d,J = 11,2 Hz, 1H); 8,15 (é, 3H); 8,72 (s,1H); 9,10 (m,1H) |
| 4bA | DMSO-$d_6$/TFA | 1,31 (d,J = 6,5 Hz, 3H); 3,70-4,70 (m,6H); 7,10-7,80 (a.c., 3H); 7,85-8,15 (m,4H); 8,71 (s,1H), 8,94 (m,1H) |
| 2bL | DMSO-$d_6$/TFA-$d_1$ | 0,87 (d,J = 5,6 Hz, 6H); 1,38 (s,12 H); 3,80-4,70 (m, 6H); 7,15-7,90 (m,3H); 8,05 (d,J = 11,3 Hz, 1H); 8,78 (s,1H) |

TABLEAU II (Cont.)

| Exemple | Solvant | RMN-$^1$H (100 $MH_z$) |
|---|---|---|
| 3bL | DMSO-$d_6$/TFA | 0,89 (d,J = 5,3 Hz, 6H); 1,57 (m,3H); 3,67 (m,1H); 4,02 (m,2H); 4,42 (m,3H); 7,19-7,86 (m,3H); 8,03 (d,J = 11,2 Hz, 1H); 8,16 (é, 3H); 8,74 (s,1H); 8,99 (m,1H) |
| 4bL | DMSO-$d_6$/TFA | 0,87 (d,J = 5,1 Hz, 6H); 1,54 (m,3H); 3,60-4,70 (a.c., 6H); 7,15-7,80 (m,3H); 8,03 (d,J = 11,2 Hz, 1H); 8,16 (é, 3H); 8,75 (s,1H); 9,35 (m,1H) |
| 2bG | DMSO-$d_6$/TFA-$d_1$ | 1,33 (s,9H); 3,49 (s,2H); 3,80-4,70 (m,5H); 7,10-7,80 (m,3H); 7,99 (d,J = 10,6 Hz, 1H); 8,73 (s,1H) |
| 3bG | DMSO-$d_6$/TFA | 3,53 (d,J = 5,9 Hz, 2H); 3,65-4,70 (a.c., 5H); 7,15-7,80 (m,3H); 7,80-8,15 (a.c., 4H (δ = 8,03, d,J = 11,3 Hz)); 8,77 (s,1H); 8,97 (m,1H) |
| 4bG | DMSO-$d_6$/TFA | 3,51 (d,J = 5,9 Hz, 2H); 3,70-4,80 (a.c., 5H); 7,05-7,80 (m,3H); 7,80-8,15 (a.c., 4H (δ = 7,98, d,J = 11,3 Hz)); 8,70 (s,1H); 9,00 (m,1H) |

TABLEAU II (Cont.)

| Exemple | Solvant | RMN-$^1$H (100 MH$_z$) |
|---|---|---|
| 2ca | DMSO-$d_6$/TFA-$d_1$ | 0,85-1,28 (a.c., 7H); 1,54 (s,3H); 3,79-4,60 (a.c., 6H); 5,00 (s,2H); 7,30 (s,5H); 7,64 (d,J = 13,1 Hz, 1H); 8,55 (s,1H) |
| 3ca | DMSO-$d_6$/TFA | 1,15 (m,4H); 1,36 (d,J = 6,5 Hz, 3H); 1,57 (s,3H); 3,65-4,70 (a.c., 6H); 7,67 (d,J = 11,4 Hz, 1H); 8,05 (é, 3H); 8,56 (s,1H); 8,90 (m,1H) |
| 4ca | DMSO-$d_6$/TFA | 1,12 (m,4H); 1,34 (d,J = 5,5 Hz, 3H); 1,55 (s,3H); 3,70-4,70 (a.c., 6H); 7,68 (d,J = 12,7 Hz, 1H); 8,01 (é, 3H); 8,55 (s,1H); 8,79 (m,1H) |
| 2da | DMSO-$d_6$/TFA-$d_1$ | 0,75-1,34 (a.c., 7H); 1,50 (s,3H); 3,84-4,64 (a.c., 6H); 4,98 (s,2H); 7,25 (s,5H); 7,69 (d,J = 12,9 Hz, 1H); 8,68 (s,1H) |
| 3da | DMSO-$d_6$/TFA | 0,90-1,20 (m,4H); 1,37 (d,J = 7,0 Hz, 3H); 1,57 (s,3H); 3,81 (m,1H); 4,10-4,70 (a.c., 5H); 7,75 (d,J = 13,7 Hz, 1H); 8,1 (m,3H); 8,72 (s,1H); 9,08 (m,1H) |

TABLEAU II (Cont.)

| Exemple | Solvant | RMN-$^1$H (100 MH$_z$) |
|---|---|---|
| 4da | DMSO-$d_6$/TFA | 0,90-1,20 (m,4H); 1,36 (d,J = 6,9 Hz, 3H); 1,56 (s,3H); 3,80 (m,1H); 4,10-4,70 (a.c., 5H); 7,75 (d,J = 13,3 Hz, 1H); 8,0 (m,3H); 8,70 (s,1H); 9,00 (m,1H) |
| 2ea | DMSO-$d_6$/TFA-$d_1$ | 1,15 (a.c., 6H); 3,60-4,45 (a.c., 5H); 4,97 (s,2H); 5,74 (d,J = 7,3 Hz, 1H); 7,10-7,80 (a.c., 8H (δ = 7,27, s, 5H)); 7,87 (d,J = 12,6,1H); 8,69 (s,1H) |
| 3ea | DMSO-$d_6$/TFA | 1,32 (a.c., 6H); 3,60-4,50 (a.c., 5H); 5,75 (d,J = 7,2 Hz, 1H); 7,10-7,60 (a.c., 3H); 7,86 (d,J = 12,7 Hz, 1H); 8,10 (é, 3H); 8,64 (s,1H); 8,75 (m,1H) |
| 4ea | DMSO-$d_6$/TFA | 1,33 (a.c., 6H); 3,60-4,50 (a.c., 5H); 5,76 (d,J = 7,2 Hz, 1H); 7,10-7,60 (a.c., 3H); 7,87 (d,J = 12,7 Hz, 1H); 8,10 (é, 3H); 8,65 (s,1H); 8,76 (m,1H) |
| 2bN | DMSO-$d_6$/TFA-$d_1$ | 0,81 (m,3H); 1,00-1,70 (a.c., 13H (δ= 1,33, s, 9H)); 3,70-4,70 (a.c., 6H); 7,10-7,85 (a.c., 3H); 8,01 (d,J = 11,3 Hz, 1H); 8,75 (s,1H) |

TABLEAU II (Cont.)

| Exemple | Solvant | RMN-$^1$H (100 MH$_z$) |
|---|---|---|
| 3bN | DMSO-$d_6$/TFA | 0,82 (m,3H); 1,28 (m,2H); 1,61 (m,2H); 3,60-4,70 (a.c., 6H); 7,10-7,85 (a.c., 3H); 7,94 (d,J = 11,3 Hz; 1H); 8,10 (m,3H); 8,70 (s,1H); 9,01 (m,1H) |
| 4bN | DMSO-$d_6$/TFA | 0,86 (t,J = 6,9 Hz, 3H); 1,22 (m,2H); 1,64 (m,2H); 3,60-4,70 (a.c., 6H); 7,10-7,85 (a.c., 3H); 8,04 (d,J = 11,3 Hz, 1H); 8,12 (m,3H); 8,75 (s, 1H); 9,11 (m,1H) |
| 2aa | DMSO-$d_6$/TFA-$d_1$ | 1,03 (m,4H); 1,19 (d,J = 7,2 Hz, 3H); 1,54 (d,J = 6,0 Hz, 3H); 3,57 (m,1H); 3,85-4,80 (a.c., 5H); 4,96 (s,2H); 7,23 (s,5H); 7,87 (d,J = 11,5 Hz, 1H); 8,53 (s,1H) |
| 3aa | DMSO-$d_6$/TFA | 1,08 (m,4H); 1,35 (d,J = 6,6 Hz, 3H); 1,59 (d,J = 6,1 Hz, 3H); 3,50-4,80 (a.c., 6H); 7,95 (d,J = 11,9 Hz, 1H); 8,11 (m,3H); 8,55 (s,1H); 8,93 (m,1H) |
| 4aa | DMSO-$d_6$/TFA | 1,07 (m,4H); 1,36 (d,J = 7,0 Hz,3H); 1,58 (d,J = 6,0 Hz, 3H); 3,50-4,80 (a.c., 6H); 7,91 (d,J = 11,5 Hz, 1H); 8,04 (m,3H); 8,54 (s,1H); 8,95 (m,1H) |

TABLEAU II (Cont.)

| Exemple | Solvant | RMN-$^1$H (100 $MH_2$) |
|---|---|---|
| 2ba | DMSO-$d_6$/TFA-$d_1$ | 1,18 (d,J = 7,0 Hz, 3H); 3,70-4,70 (a.c., 6H); 4,99 (s,2H); 7,10-7,80 (a.c., 8H, (δ = 7,28, s, 5H)); 7,98 (d,J = 11,3 Hz,1H); 8,71 (s,1H) |
| 3ba | DMSO-$d_6$/TFA | 1,32 (d,J = 7,0 Hz, 3H); 3,70-4,15 (m,3H); 4,15-4,70 (m,3H); 7,10-7,80 (a.c., 3H); 8,00 (d,J = 11,1 Hz, 1H); 8,13 (é, 3H); 8,71 (s,1H); 9,08 (m,1H) |
| 4ba | DMSO-$d_6$/TFA | 1,31 (d,J = 6,5 Hz, 3H); 3,70-4,70 (m,6H); 7,10-7,80 (a.c., 3H); 7,85-8,15 (m,4H); 8,70 (s,1H); 8,93 (m,1H) |
| 2fa | DMSO-$d_6$/TFA-$d_1$ | 1,12 (m,4H); 1,22 (d,J = 7,0 Hz, 3H); 1,60 (d,J = 6,1 Hz, 3H); 3,61 (m,1H); 3,85-4,70 (a.c., 5H); 5,02 (s,2H); 7,31 (s,5H); 7,98 (d,J = 11,5 Hz,1H); 8,57 (s,1H) |
| 3fa | DMSO-$d_6$/TFA | 1,16 (m,4H); 1,39 (d,J = 7,0 Hz, 3H); 1,65 (d,J = 6,2 Hz, 3H); 3,55-4,00 (m,2H); 4,00-4,80 (a.c., 4H); 8,02 (d,J = 11,6 Hz, 1H); 8,15 (é, 3H); 8,60 (s,1H); 8,95 (m,1H) |
| 4fa | DMSO-$d_6$/TFA | 1,11 (m,4H); 1,39 (d,J = 7,0 Hz, 3H); 1,64 (d,J = 6,2 Hz, 3H); 3,50-4,00 (m,2H); 4,00-4,80 (a.c., 4H); 8,01 (d,J = 11,5 Hz, 1H); 8,17 (é, 3H); 8,59 (s,1H); 8,97 (m,1H) |

TABLEAU II (Cont.)

| Exemple | Solvant | RMN-$^1$H (100 MHz) |
|---|---|---|
| 2aAA | DMSO-$d_6$/TFA-$d_1$ (300 MHz) | 0,90-1,26 (m,13H); 1,56 (d,J = 6 Hz, 3H); 3,55 (m,1H); 3,98 (m,1H); 4,2 (m,3H); 4,45 (m,1H); 4,58 (m,1H); 4,78 et 4,85 (système AB,J = 12,0 Hz, 2H); 7,87 (d,J = 12,0 Hz, 1H); 8,47 (s,1H) |
| 3eAA | DMSO-$d_6$/TFA | 0,9-1,4 (m,13H); 1,60 (d,J = 5,74 Hz, 3H); 3,5-4,8 (m,7H); 7,85-8,20 (m,4H (δ = 8,00, d,J = 11,2 Hz)); 8,56 (s,1H); 8,63 (é,2H) |
| 4eAA | DMSO-$d_6$/TFA | 0,9-1,5 (m,13H); 1,61 (d,J = 6,2 Hz, 3H); 3,5-4,7 (m,7H); 7,88 (d,J = 11,2 Hz, 1H); 8,05 (m,3H); 8,4-8,6 (m,3H (δ = 8,52, s)) |
| 2eA | DMSO-$d_6$/TFA-$d_1$ | 1,20 (m,6H); 3,60-4,40 (m,5H); 4,97 (s,2H); 5,74 (d,J = 6,7 Hz, 1H); 7,10-8,00 (m,9H (δ = 7,26, s),(δ = 7,88, d,J = 11,56 Hz)); 8,71 (s,1H) |
| 3eA | DMSO-$d_6$/TFA | 1,28 (m,6H); 2,40 (m,2H); 4,15 (m,3H) 5,74 (d,J = 7,0 Hz, 1H); 7,10-8,3 (m,7H (δ = 7,87, d,J = 12,7 Hz)); 8,65 (s,1H); 8,75 (m,1H) |
| 4eA | DMSO-$d_6$/TFA | 1,26 (m,6H); 2,21 (s,3H); 5,71 (d,J = 7,3 Hz, 1H); 7,00-8 (m,11H, (δ = 7,08 et 7,51, système AB,J = 8,2 Hz),(δ = 7,85, d,J = 13.0 Hz)); 8,60 (s,1H); 8,75 (m,1H) |

TABLEAU II (Cont.)

| Exemple | Solvant | RMN-$^1$H (100 MHz) |
|---|---|---|
| 2eN | DMSO-$d_6$/TFA-$d_1$ | 0,77 (m,3H); 0,95-1,60 (m,16H,(δ = 1,28, s)); 3,75 (m,2H); 4,1 (m,3H); 5,72 (d,J = 7,0 Hz, 1H); 7,10-7,95 (m,4H,(δ = 7,83, d,J = 12,6 Hz)); 8,61 (s,1H) |
| 3eN | DMSO-$d_6$/TFA | 7,77 (m,3H); 1,23 (m,5H); 1,64 (m,2H) 3,59 (m,2H); 4,11 (m,3H); 5,68 (d,J = 7,0 Hz, 1H); 7,00-8,10 (m,6H,(δ = 7,72, d,J = 12.0 Hz)); 8,58 (s,1H); 8,74 (m,1H) |
| 4eN | DMSO-$d_6$ (300 MHz) | 0,86 (t,J = 7,6 Hz, 3H); 1,29 (m,5H); 1,63 (m,2H); 2,28 (s,3H); 3,60-3,80 (m,2H); 4,00-4,20 (m,2H); 4,40 (m,1H) 5,75 (d,J = 7,0 Hz, 1H); 7,09 et 7,47 (système AB,J = 8,0 Hz, 4H); 7,43 (m,1H); 7,74 (m,1H); 7,85-8,00 (m,2H, (δ = 7,95, d,J = 12,0 Hz)); 8,05 (é, 2,H); 8,79 et 8,81 (2 x s, 1H); 8,93 (m,1H) |

## Solubilité

Un grand nombre des composés de l'invention manifestent une augmentation de la solubilité dans l'eau ainsi que de la solubilité à pH 7,4 lorsqu'on compare avec les composés de référence. L'augmentation de solubilité au pH physiologique peut favoriser l'absorption du produit et la solubilité élevée dans l'eau permet l'administration du composé par voie parentérale.

| Exemple | A | Sel | Solubilité dans l'eau (µg/ml) | Solubilité pH 7,4 (µg/ml) |
|---|---|---|---|---|
| 1a | H | - | 23,0 | 16,5 |
| 4aA | Ala | HCl | >500 | >500 |
| 4aL | Leu | HCl | >500 | >500 |
| 1b | H | - | 3,0 | 3,7 |
| 4bA | Ala | HCl | 128,4 | 66,9 |
| 4bG | Gly | HCl | >500 | 7,6 |
| 4bL | Leu | HCl | >500 | 23,1 |
| 4aa | D-Ala | HCl | 494,9 | 385,6 |
| 4fa | D-Ala | HCl | >500 | >500 |
| 4aAA | Ala-Ala | HCl | >500 | >500 |
| 4eA | Ala | TsOH | >500 | >500 |
| 4eN | Nval | TsOH | >500 | >500 |

ACTIVITE BIOLOGIQUE

L'activité pharmacologique antimicrobienne de ces composés a été étudiée selon les références exposées ci-dessous.

Activité pharmacologique antimicrobienne : G.L. Daquet et Y.A. Chabbect, techniques en bactériologie, Vol. 3, Flammarion Médecine-Sciences, Paris 1972, et W.B. Hugo et A.D. Rusell, Pharmaceutical Microbiology, Blackwell Scientific Publications, London, 1977.

Milieu de culture et solvant :

Agar d'antibiotiques n° 1 (Oxoid CM 327)
Bouillon de triptone-soja (Oxoid CM 129)
Solution Physiologique Ringer 1/4 (Oxoid BR 52)
Agar Dextrose (BBL 11165)

Microorganismes :

"Bacillus subtilis" ATCC 6633
"Citrobacter freundii" ATCC 112606
"Enterobacter aerogenes" ATCC 15038
"Enterobacter cloacae" ATCC 23355
"Bacillus cereus" ATCC 1178
"Escherichia coli" ATCC 10799
"Escherichia coli" ATCC 23559
"Klebsiella pneumoniae" ATCC 10031

"Proteus vulgaris" ATCC 8427
"Morg. morganii" ATCC 8019
"Pseudomonas aeruginosa" ATCC 9721
"Pseudomonas aeruginosa" ATCC 10145
"Salmonella typhimurium" ATCC 14028
"Salmonella typhimurium" ATCC 6539
"Serratia marcescens" ATCC 13880
"Shigella flexnerii" ATCC 12022
"Staphylococcusepidermis" ATCC 155-1
"Staphylococcus aureus" ATCC 25178
"Streptococcus faecalis" ATCC 10541.

Préparation des inoculations

Chacun des microorganismes est ensemencé par strie dans des tubes d'Agar d'antibiotiques n° 1, et mis en incubation pendant 20 heures à 37°C. Ensuite, on prend une anse de culture, on ensemence dans un bouillon de Triptone-soja et on incube pendant 20 heures à 37°C. On dilue à 1/4 la culture obtenue avec une solution physiologique Ringer, de façon à obtenir une suspension normalisée de $10^{-10}$ ufc/ml pour chaque organisme.

Préparation du milieu contenant les dérivés de formule générale I :

A partir d'une solution de 100 µg/ml, chaque produit est dilué dans l'Agar Dextrose (préalablement fondu et maintenu à 50°C) par dilutions successives de façon à obtenir les concentrations suivantes : 64-32-16-8-4-2-1-0,5-0,25-0,125 µg de dérivé/ml du milieu.

Postérieurement, chaque concentration de chaque produit est répartie dans des boîtes de Petri de 10 cm de diamètre, à raison de 10 ml de milieu par boîte et autant de boîtes que de microorganismes à tester.

Une fois le milieu refroidi, les boîtes sont ensemencées avec les inoculations à raison de 0,4 ml d'inoculation par boîte. On les étend avec une anse de Driglasky et on recueille le surnageant. Les boîtes ensemencées sont incubées à 37°C pendant 20 heures.

Les résultats obtenus sont décrits dans les tableaux suivants. Les concentrations sont données en µg/ml.

| MICROORGANISMES | EXEMPLES | | | | |
|---|---|---|---|---|---|
| | Acide pipémidique | 4aA | 4aL | 4bA | 4bL |
| Bacillus subtilis ATCC 6633 | 8 | 0,12 | 0,12 | 0,5 | 0,25 |
| Bacillus cereus ATCC 11778 | 16 | 1 | 0,12 | 4 | 0,5 |
| Strep. faecalis ATCC 10541 | >64 | 8 | 2 | 8 | 1 |
| Staph. aureus ATCC 25178 | 64 | 0,5 | 0,25 | 1 | 4 |
| Staph. epidermidis ATCC 155-1 | 64 | 0,5 | 0,25 | 4 | 1 |
| Ps. aeruginosa ATCC 9721 | 32 | 4 | 2 | 8 | 4 |
| Ps. aeruginosa ATCC 10145 | 32 | 8 | 8 | 16 | 8 |
| Citr. freundii ATCC 11606 | 4 | 0,25 | 0,12 | 1 | 1 |
| Morg. morganii ATCC 8019 | 8 | 0,5 | 0,25 | 2 | 2 |
| Proteus vulgaris ATCC 8427 | 16 | 1 | 0,25 | 8 | 2 |
| Kleb. pneumoniae ATCC 10031 | 2 | 0,06 | 0,06 | 1 | 0,12 |
| Sal. typhimurium ATCC 14028 | 8 | 0,5 | 0,12 | 2 | 1 |
| Sal. typhi ATCC 6539 | 4 | 0,5 | 0,12 | 1 | 1 |
| Escherichia coli ATCC 10799 | 16 | 0,5 | 0,12 | 2 | 1 |
| Escherichia coli ATCC 23559 | 2 | 0,12 | 0,06 | 1 | 0,25 |
| Ent. aerogenes ATCC 15038 | 32 | 0,5 | 0,12 | 1 | 0,5 |
| Ent. cloacae ATCC 23355 | 8 | 0,12 | 0,12 | 1 | 0,5 |
| Serr. marcescens ATCC 13880 | 16 | 1 | 0,25 | 8 | 2 |
| Shigella flexnerii ATCC 12022 | 4 | 0,12 | 0,06 | 1 | 0,25 |

| MICROORGANISMES | EXEMPLES | | | |
|---|---|---|---|---|
| | 4bG | 4ca | 4bN | 4aa |
| Bacillus subtilis ATCC 6633 | 1 | 0,25 | 0,25 | 0,12 |
| Bacillus cereus ATCC 11778 | 1 | 1 | 0,5 | 0,5 |
| Strep. faecalis ATCC 10541 | 16 | 8 | 4 | 1 |
| Staph. aureus ATCC 25178 | 1 | 0,5 | 4 | 0,25 |
| Staph. epidermidis ATCC 155-1 | 2 | 0,5 | 2 | 1 |
| Ps. aeruginosa ATCC 9721 | 16 | 4 | 4 | 4 |
| Ps. aeruginosa ATCC 10145 | >32 | 16 | 16 | 8 |
| Citr. freundii ATCC 11606 | 1 | 1 | 1 | 0,5 |
| Morg. morganii ATCC 8019 | 4 | 1 | 2 | 2 |
| Proteus vulgaris ATCC 8427 | 8 | 2 | 4 | 2 |
| Kleb. pneumoniae ATCC 10031 | 1 | 0,25 | 0,12 | 0,5 |
| Sal. typhimurium ATCC 14028 | 4 | 1 | 1 | 1 |
| Sal. typhi ATCC 6539 | 1 | 0,5 | 1 | 0,5 |
| Escherichia coli ATCC 10799 | 2 | 1 | 2 | 1 |
| Escherichia coli ATCC 23559 | 0,5 | 0,5 | 0,25 | 0,25 |
| Ent. aerogenes ATCC 15038 | 1 | 1 | 1 | 0,5 |
| Ent. cloacae ATCC 23355 | 1 | 0,5 | 0,5 | 0,25 |
| Serr. marcescens ATCC 13880 | 16 | 2 | 2 | 4 |
| Shigella flexnerii ATCC 12022 | 0,5 | 0,25 | 0,12 | 0,12 |

| MICROORGANISMES | EXEMPLES | | | |
|---|---|---|---|---|
| | 4fa | 4aAA | 4eA | 4eN |
| Bacillus subtilis ATCC 6633 | 0,5 | 1 | 0,5 | 0,25 |
| Bacillus cereus ATCC 11778 | 1 | 16 | 1 | 0,5 |
| Strep. faecalis ATCC 10541 | 1 | >16 | 8 | 4 |
| Staph. aureus ATCC 25178 | 1 | 4 | 1 | 0,5 |
| Staph. epidermidis ATCC 155-1 | 0,25 | 8 | 16 | 0,5 |
| Ps. aeruginosa ATCC 9721 | >16 | >16 | 8 | 4 |
| Ps. aeruginosa ATCC 10145 | >16 | >16 | 8 | 16 |
| Citr. freundii ATCC 11606 | 4 | 2 | 2 | 2 |
| Morg. morganii ATCC 8019 | 8 | 4 | 4 | 4 |
| Proteus vulgaris ATCC 8427 | 8 | 8 | 16 | 8 |
| Kleb. pneumoniae ATCC 10031 | 8 | 8 | 2 | 0,25 |
| Sal. typhimurium ATCC 14028 | 4 | 2 | 2 | 1 |
| Sal. typhi ATCC 6539 | 4 | 2 | 2 | 0,5 |
| Escherichia coli ATCC 10799 | 4 | 2 | 2 | 0,5 |
| Escherichia coli ATCC 23559 | 1 | 2 | 1 | 0,5 |
| Ent. aerogenes ATCC 15038 | 4 | 2 | 2 | 2 |
| Ent. cloacae ATCC 23355 | 4 | 2 | 2 | 1 |
| Serr. marcescens ATCC 13880 | 16 | 8 | 8 | 16 |
| Shigella flexnerii ATCC 12022 | 1 | 2 | 0,5 | 0,5 |

En thérapeutique humaine, la dose d'administration est, bien sûr, fonction de la susceptibilité de la souche infective, de la nature de composé administré et de la voie d'administration. Elle sera généralement comprise entre environ 0,200 et environ 300 mg pour chaque kilogramme de poids et par jour. Les dérivés de l'invention seront, par exemple, administrés sous forme de comprimés, de solutions ou de suspensions, ou bien de gélules.

On indiquera ci-après, à titre d'exemples, deux formes galéniques particulières des dérivés objets de la présente invention.

| Exemple de formule par comprimé | |
|---|---|
| Composé de l'exemple 4aL | 250 mg |
| Cellule microcristalline | 69 mg |
| Povidone | 15 mg |
| Amidon de blé | 36 mg |
| Dioxyde de silice colloïdale | 2 mg |
| Stéarate de magnésium | 3 mg |
| Poids comprimé | 375 mg |

| Exemple de formule par gélule | |
|---|---|
| Composé de l'exemple 4aL | 250 mg |
| Glycéride polyoxyéthylénée | 85 mg |
| Behenate de glycérine | 15 mg |
| Excipiente gélatine molle q.s. | 450 mg |

| Exemple de formule par injectable | |
|---|---|
| Composé de l'exemple 4aL | 100 mg |
| HCl 1M q.s. | pH 3-4 |
| $H_2O$ pour injection q.s. | 10 ml |

## Revendications

1. Les composés répondant à la formule générale I

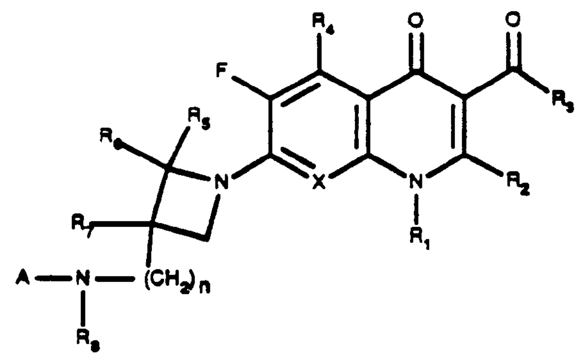

I

et leurs sels pharmaceutiquement acceptables, formule dans laquelle

X représente un atome d'azote ou bien un atome de carbone lié à un atome d'hydrogène (C-H) ou bien un atome de carbone lié à un atome d'halogène (C-Y) dans lequel Y représente un atome de fluor, de chlore ou de brome, ou bien un atome de carbone lié à un radical hydroxy (C-OH) ou alkyloxy (C-O-alkyle), ou bien un atome de carbone lié à un radical alkyle (C-alkyle), ou bien un atome de carbone lié à un radical alkyle halogéné (C-$CF_3$), ou bien un atome de carbone lié à un radical alkylamino (C-NH-alkyle).

$R_1$ représente un radical alkyle ou cycloalkyle inférieur, un radical halogénoalkyle inférieur, un radical hydroxyalkyle, un radical vinyle, un radical aryle ou un radical aryle substitué par un ou plus atomes de fluor, ou un radical alkylamino (NH-alkyle).

$R_2$ représente un atome d'hydrogène ou un radical alkyle inférieur.

$R_3$ représente un radical hydroxy ou un radical alkyloxy inférieur de $C_1$ à $C_4$.

$R_4$ représente un atome d'hydrogène, un atome de fluor, un radical alkyle inférieur, un radical nitro, un radical amino ou un radical amino substitué.

$R_5$, $R_6$ et $R_7$ représentent un atome d'hydrogène ou un radical alkyle inférieur. Les substituants azétidiniques peuvent avoir, selon la nature et la position relative, jusqu'à deux centres chiraux, chacun d'eux avec une configuration "R" ou "S".

$R_8$ représente un atome d'hydrogène ou un radical alkyle inférieur.

n est un nombre qui peut être 0 ou 1.

X et $R_1$ peuvent former ensemble une liaison représentée par un groupe C-$CH_2$-$CH_2$-CH-$R_9$ ou C-O-$CH_2$-CH-$R_9$ ou C-S-$CH_2$-CH-$R_9$ dans lesquels $R_9$ représente un atome d'hydrogène, un radical alkyle inférieur ou un radical halogénoalkyle inférieur et possédant dans ces deux derniers cas un centre chiral avec une configuration "R" ou "S".

$R_1$ et $R_2$ peuvent former ensemble une liaison représentée par un groupe -$CHR_{10}$-S- dans lequel $R_{10}$ représente un atome d'hydrogène ou un radical alkyle inférieur ou un radical halogénoalkyle inférieur et possédant dans ces deux derniers cas un centre chiral avec une configuration "R" ou "S".

$R_2$ et $R_3$ peuvent former ensemble une liaison représentée par un groupe -S-NH-.

A est un groupe solubilisateur qui est représenté par un aminoacide résiduel ou une chaîne polypeptidique constituée de deux à quatre ou plus résidus d'aminoacides, lesquels sont unis de façon covalente entre eux au travers de liaisons peptidiques.

Les résidus d'aminoacides de la présente invention comprennent les 20 aminoacides naturels, désignés moyennant leurs trois lettres symboliques, ainsi que d'autres aminoacides, notamment la norvaline (Nva), homosérine, 4-hydroxyproline, hydroxylysine, ornitine.

La configuration stéréochimique des aminoacides de la présente invention peuvent être de la forme D- ou L- ou un mélange des deux, étant préférable, nonobstant, l'utilisation de résidus d'aminoacides sous forme L-.

2. Les composés répondant à la formule générale I, selon la revendication 1, sélectionnés parmi le groupe suivant :

* Acide 7-[(2S,3R)-2-méthyl-3-N-CBZ-Ala-amino-1-azétidinyl]-1-cyclopropyl-6-fluoro-1,4-dihydro-4-oxo-1,8-naphtyridine -3-carboxylique.

* Acide 7-[(2S,3R)-2-méthyl-3-Ala-amino-1-azétidinyl]-1-cyclopropyl -6-fluoro-1,4-dihydro-4-oxo-1,8-naphtyridine-3-carboxylique.

∗ Chlorhydrate de l'acide 7-[(2S,3R)-2-méthyl-3-Ala-amino-1-azétidinyl]-1-cyclopropyl-6-fluoro-1,4-dihydro-4-oxo-1,8-naphtyridine-3-carboxylique.

∗ Acide 7-[(2S,3R)-2-méthyl-3-N-t-BOC-Leu-amino-1-azétidinyl]-1-cyclopropyl-6-fluoro-1,4-dihydro-4-oxo-1,8-naphtyridine-3-carboxylique.

∗ Acide 7-[(2S,3R)-2-méthyl-3-Leu-amino-1-azétidinyl]-1-cyclopropyl-6-fluoro-1,4-dihydro-4-oxo-1,8-naphtyridine-3-carboxylique.

∗ Chlorhydrate de l'acide 7-[(2S,3R)-2-méthyl-3-Leu-amino-1-azétidinyl]-1-cyclopropyl-6-fluoro-1,4-dihydro-4-oxo-1,8-naphtyridine-3-carboxylique.

∗ Acide 7-(3-N-CBZ-Ala-amino-1-azétidinyl)-1-(2,4-difluorophényl)-6-fluoro-1,4-dihydro-4-oxo-1,8-naphtyridine-3-carboxylique.

∗ Acide 7-(3-Ala-amino-1-azétidinyl)-1-(2,4-difluorophényl)-6-fluoro-1,4-dihydro-4-oxo-1,8-naphtyridine-3-carboxylique.

∗ Chlorhydrate de l'acide 7-(3-Ala-amino-1-azétidinyl)-1-(2,4-difluorophényl)-6-fluoro-1,4-dihydro-4-oxo-1,8-naphtyridine-3-carboxylique.

∗ Acide 7-(3-N-t-BOC-Leu-amino-1-azétidinyl)-1-(2,4-difluorophényl)-6-fluoro-1,4-dihydro-4-oxo-1,8-naphtyridine-3-carboxylique.

∗ Acide 7-(3-Leu-amino-1-azétidinyl)-1-(2,4-difluorophényl)-6-fluoro-1,4-dihydro-4-oxo-1,8-naphtyridine-3-carboxylique.

∗ Chlorhydrate de l'acide 7-(3-Leu-amino-1-azétidinyl)-1-(2,4-difluorophényl)-6-fluoro-1,4-dihydro-4-oxo-1,8-naphtyridine-3-carboxylique.

∗ Acide 7-(3-N-t-BOC-Gly-amino-1-azétidinyl)-1-(2,4-difluorophényl)-6-fluoro-1,4-dihydro-4-oxo-1,8-naphtyridine-3-carboxylique.

∗ Acide 7-(3-Gly-amino-1-azétidinyl)-1-(2,4-difluorophényl)-6-fluoro-1,4-dihydro-4-oxo-1,8-naphtyridine-3-carboxylique.

∗ Chlorhydrate de l'acide 7-(3-Gly-amino-1-azétidinyl)-1-(2,4-difluorophényl)-6-fluoro-1,4-dihydro-4-oxo-1,8-naphtyridine-3-carboxylique.

∗ Acide 7-(3-méthyl-3-N-CBZ-D-Ala-amino-1-azétidinyl)-1-cyclopropyl-6,8-difluoro-1,4-dihydro-4-oxo-3-quinoléinecarboxylique.

∗ Acide 7-(3-méthyl-D-3-Ala-amino-1-azétidinyl)-1-cyclopropyl-6,8-difluoro-1,4-dihydro-4-oxo-3-quinoléinecarboxylique.

∗ Chlorhydrate de l'acide 7-(3-méthyl-3-D-Ala-amino-1-azétidinyl)-1-cyclopropyl-6,8-difluoro-1,4-dihydro-4-oxo-3-quinoléinecarboxylique.

∗ Acide 7-(3-méthyl-3-N-CBZ-D-Ala-amino-1-azétidinyl)-1-cyclopropyl-6-fluoro-8-chloro-1,4-dihydro-4-oxo-3-quinoléinecarboxylique.

∗ Acide 7-(3-méthyl-3-D-Ala-amino-1-azétidinyl)-1-cyclopropyl-6-fluoro-8-chloro-1,4-dihydro-4-oxo-3-quinoléinecarboxylique.

∗ Chlorhydrate de l'acide 7-(3-méthyl-3-D-Ala-amino-1-azétidinyl)-1-cyclopropyl-6-fluoro-8-chloro-1,4-dihydro-4-oxo-3-quinoléinecarboxylique.

∗ Acide 7-[(2S,3R)-2-méthyl-3-N-CBZ-D-Ala-amino-1-azétidinyl]-1-(2,4-difluorophényl)-6-fluoro-1,4-dihydro-4-oxo-3-quinoléinecarboxylique.

∗ Acide 7-[(2S,3R)-2-méthyl-3-D-Ala-amino-1-azétidinyl]-1-(2,4-difluorophényl)-6-fluoro-1,4-dihydro-4-oxo-3-quinoléinecarboxylique.

∗ Chlorhydrate de l'acide 7-[(2S,3R)-2-méthyl-3-D-Ala-amino-1-azétidinyl]-1-(2,4-difluorophényl)-6-fluoro-1,4-dihydro-4-oxo-3-quinoléinecarboxylique.

∗ Acide 7-(3-N-t-BOC-Nva-amino-1-azétidinyl)-1-(2,4-difluorophényl)-6-fluoro-1,4-dihydro-4-oxo-1,8-naphtyridine-3-carboxylique.

∗ Acide 7-(3-Nva-amino-1-azétidinyl)-1-(2,4-difluorophényl)-6-fluoro-1,4-dihydro-4-oxo-1,8-naphtyridine-3-carboxylique.

∗ Chlorhydrate de l'acide 7-(3-Nva-amino-1-azétidinyl)-1-(2,4-difluorophényl)-6-fluoro-1,4-dihydro-4-oxo-1,8-naphtyridine-3-carboxylique.

∗ Acide 7-[(2S,3R)-2-méthyl-3-N-CBZ-D-Ala-amino-1-azétidinyl]-1-cyclopropyl-6-fluoro-1,4-dihydro-4-oxo-1,8-naphtyridine-3-carboxylique.

∗ Acide 7-[(2S,3R)-2-méthyl-3-D-Ala-amino-1-azétidinyl]-1-cyclopropyl-6-fluoro-1,4-dihydro-4-oxo-1,8-naphtyridine-3-carboxylique.

∗ Chlorhydrate de l'acide 7-[(2S,3R)-2-méthyl-3-D-Ala-amino-1-azétidinyl]-1-cyclopropyl-6-fluoro-1,4-dihydro-4-oxo-1,8-naphtyridine-3-carboxylique.

∗ Acide 7-(3-N-CBZ-D-Ala-amino-1-azétidinyl)-1-(2,4-difluorophényl)-6-fluoro-1,4-dihydro-4-oxo-1,8-naphtyridine-3-carboxylique.

* Acide 7-(3-D-Ala-amino-1-azétidinyl)-1-(2,4-difluorophényl)-6-fluoro-1,4-dihydro-4-oxo-1,8-naphtyridine-3-carboxylique.

* Chlorhydrate de l'acide 7-(3-D-Ala-amino-1-azétidinyl)-1-(2,4-difluorophényl)-6-fluoro-1,4-dihydro-4-oxo-1,8-naphtyridine -3-carboxylique.

■ Acide 7-[(2S,3R)-2-méthyl-3-N-CBZ-Ala-Ala-amino-1-azétidinyl]-1-cyclopropyl-6-fluoro-1,4-dihydro-4-oxo-1,8-naphtyridine-3-carboxylique.

■ Acide 7-[(2S,3R)-2-méthyl-3-Ala-Ala-amino-1-azétidinyl]-1-cyclopropyl-6-fluoro-1,4-dihydro-4-oxo-1,8-naphtyridine-3-carboxylique.

■ Chlorhydrate de l'acide 7-[(2S,3R)-2-méthyl-3-Ala-Ala-amino-1-azétidinyl]-1-cyclopropyl-6-fluoro-1,4-dihydro-4-oxo-1,8-naphtyridine-3-carboxylique.

■ Acide 7-[(2S,3R)-2-méthyl-3-N-CBZ-Ala-amino-1-azétidinyl]-1-(2,4-difluorophenyl)-6-fluoro-1,4-dihydro-4-oxo-3-quinoléincarboxylique.

■ Acide 7-[(2S,3R)-2-méthyl-3-Ala-amino-1-azétidinyl]-1-(2,4-difluorophenyl)-6-fluoro-1,4-dihydro-4-oxo-3-quinoléincarboxylique.

■ p-toluènesulfonate de l'acide 7-[(2S,3R)-2-méthyl-3-Ala-amino-1-azétidinyl]-1-(2,4-difluorophenyl)-6-fluoro-1,4-dihydro-4-oxo-3-quinoléincarboxylique.

■ Acide 7-[(2S,3R)-2-méthyl-3-N-BOC-Nval-amino-1-azétidinyl]-1-(2,4-difluorophenyl)-6-fluoro-1,4-dihydro-4-oxo-3-quinoléincarboxylique.

■ Acide 7-[(2S,3R)-2-méthyl-3-Nval-amino-1-azétidinyl]-1-(2,4-difluorophenyl)-6-fluoro-1,4-dihydro-4-oxo-3-quinoléincarboxylique.

■ p-toluènesulfonate de l'acide 7-[(2S,3R)-2-méthyl-3-Nval-amino-1-azétidinyl]-1-(2,4-difluorophenyl)-6-fluoro-1,4-dihydro-4-oxo-3-quinoléincarboxylique.

**3.** Procédé de préparation des dérivés de formule I, selon les revendications 1 et 2, caractérisé par l'utilisation d'une des méthodes suivantes :

3.1.- Par réaction d'un composé de formule générale II

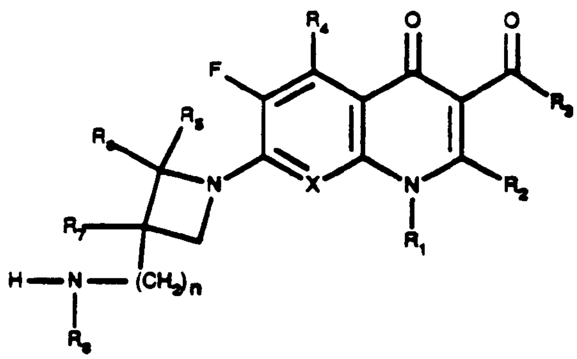

II

dans laquelle $R_1$, $R_2$, $R_3$, $R_4$, $R_5$, $R_6$, $R_7$, $R_8$, $R_9$, $R_{10}$, X et n ont les significations mentionnées précédemment, avec un composé de formule générale P1-A-P2, dans laquelle A a les significations mentionnées précédemment et P1 représente n'importe lequel des groupes protecteurs de groupes fonctionnels utilisés dans la synthèse des peptides, et P2 représente n'importe lequel des groupes activateurs de la fonction carboxylique utilisés dans la synthèse des peptides, on obtient un composé de formule générale III

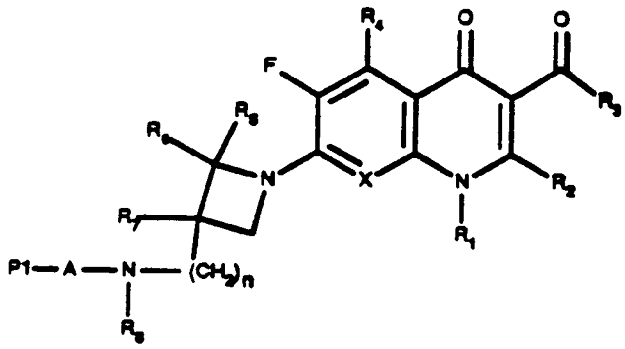

III

dans laquelle $R_1$, $R_2$, $R_3$, $R_4$, $R_5$, $R_6$, $R_7$, $R_5$, $R_9$, $R_{10}$, X, n, A et P, ont les significations mentionnées précédemment.

Le composé de formule générale III peut se former également par réaction de II avec un composé de formule générale P1-A et avec l'aide de réactifs de couplage bien connus pour la formation de liaisons peptidiques.

3.2.- Par réaction d'un composé de formule générale III, dans laquelle $R_1$, $R_2$, $R_3$, $R_4$, $R_5$, $R_6$, $R_7$, $R_8$, $R_9$, $R_{10}$, X, A, P1 et n ont les significations mentionnées précédemment avec des réactifs utiles pour l'élimination de groupes protecteurs de groupes fonctionnels présents dans les aminoacides et peptides du composé de formule générale III, on réussit la préparation du composé de formule générale I, dans laquelle $R_1$, $R_2$, $R_3$, $R_4$, $R_5$, $R_6$, $R_7$, $R_5$, $R_9$, $R_{10}$, X, A et n ont les significations mentionnées précédemment.

3.3.- Par réaction d'un composé de formule générale VIII

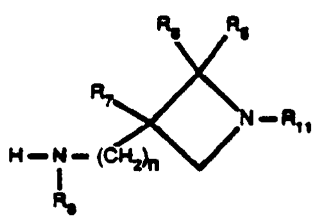

VIII

dans laquelle $R_{11}$ représente un radical diphénylméthyle et $R_5$, $R_5$, $R_7$, $R_8$ et n ont les significations mentionnées précédemment avec un composé de formule générale P1-A-P2, ou un composé de formule générale P1-A dans lequel P1, A et P2 ont les significations mentionnées précédemment et en utilisant les techniques de formation de liaisons peptidiques, on obtient un composé de formule générale VII

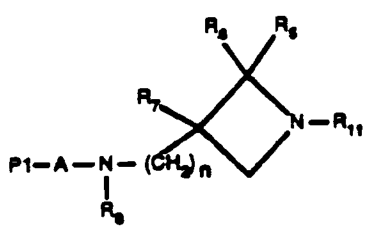

VII

dans laquelle $R_5$, $R_6$, $R_7$, $R_5$, $R_{11}$, A, P et n ont les significations mentionnées précédemment.

Par élimination des groupes protecteurs $R_{11}$ et P1, d'un composé de formule générale VII, on obtient des composés de formule générale VI

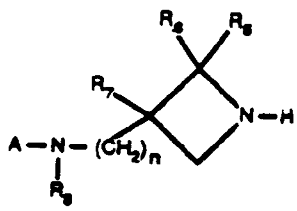

VI

dans laquelle $R_5$, $R_6$, $R_7$, $R_8$, A et n ont les significations mentionnées précédemment.

Par réaction d'un composé de formule générale IV

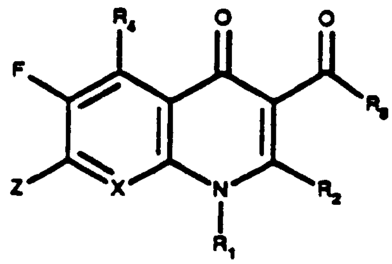

IV

dans laquelle $R_1$, $R_2$, $R_3$, $R_4$, $R_9$, $R_{10}$ et X ont les significations mentionnées précédemment et Z représente un atome d'halogène, de préférence fluor ou chlore, avec une azétidine de formule générale VI, on obtient les composés de formule générale I.

3.4.- Par hydrogénolyse d'un composé de formule générale VII, dans laquelle $R_5$, $R_6$, $R_7$, $R_8$, A, P1, n et $R_{11}$ ont les significations mentionnées précédemment, moyennant l'utilisation d'un catalyseur de palladium, de préférence Pd $(OH)_2/C$ sous atmosphère d'hydrogène et dans le sein d'un solvant approprié tel qu'un alcool, comme par exemple l'éthanol ou le méthanol, et à une pression d'hydrogène comprise entre 1 atm. et 20 atm. et les températures appropriées oscillent entre 20°C et 70°C, on obtient les composés de formule générale V

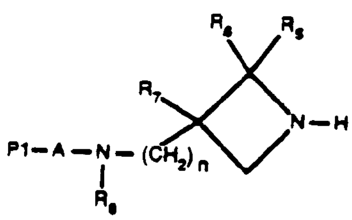

V

dans laquelle $R_5$, $R_6$, $R_7$, $R_8$, P et n ont les significations mentionnées précédemment.

Par réaction d'un composé de formule générale IV, dans laquelle $R_1$, $R_2$, $R_3$, $R_4$, $R_9$, $R_{10}$ et X ont les significations mentionnées précédemment, et Z représente un atome d'halogène, de préférence fluor ou chlore, avec une azétidine de formule générale V, on obtient les composés de formule générale III.

3.5.- Par réaction d'un composé de formule générale I avec un acide minéral ou organique dans le sein d'un solvant approprié tel que l'éthanol ou le méthanol, on obtient le sel correspondant.

**4.** Utilisation des dérivés de formule générale I et leurs sels physiologiquement acceptables, selon l'une des revendications 1 à 2, pour la fabrication de médicaments destinés au traitement des infections microbiennes, particulièrement pour la prophylaxie et la chimiothérapie des infections locales et systémiques produites par des paghogènes aérobes ou anaérobes tant Gram-positifs comme Gram-négatifs en médecine humaine ou vétérinaire.

5. Utilisation selon la revendication 4, caractérisée en ce que le médicament est destiné à être utilisé par voie parentérale.

6. Utilisation des dérivés de formule générale I et leurs sels thérapeutiquement acceptables, selon les revendications 1 à 2, comme pro-drogues des azétidinylquinolones, azétidinyl-naphtyridines, azétidinylpyridobenzoxazines, azétidinylisothiazolo-quinolones, azétidinylisothiazolo-naphtyridines ou azétidinylisothiazolopyrido-benzoxazines préalablement connus, étant donné que le groupe solubilisateur A, lorsque A est un résidu d'aminoacide ou une chaîne polypeptidique, peut souffrir une hydrolyse enzymatique qui produit la libération des azétidinylquinolones, des azétidinylnaphtyridines, des azétidinyl-pyrido-benzoxazines, des azétidinylisothiazolo-quinolones, des azétidinylisothiazolopyrido-benzoxazines préalablement connus.

7. A titre de produits intermédiaires, les composés représentés par les formules III, V, VI et VII dans lesquelles $R_1$, $R_2$, $R_3$, $R_4$, $R_5$, $R_6$, $R_7$, $R_8$, $R_9$, $R_{10}$, $R_{11}$, n, A et P ont les significations mentionnées précédemment.

Office européen des brevets

# RAPPORT DE RECHERCHE EUROPEENNE

Numero de la demande

EP 92 40 1318

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5 ) |
| --- | --- | --- | --- |
| X | EP-A-0 360 258 (ABBOTT LABORATORIES)<br>* page 2, ligne 15 - ligne 30; revendications 1-12; exemples *<br>--- | 1,4-6 | C07D471/04<br>C07D401/04<br>C07D205/04<br>C07D215/56<br>A61K31/47 |
| X | EP-A-0 304 087 (WARNER-LAMBERT COMPANY)<br>* page 2, ligne 1 - ligne 7 *<br>* page 3, ligne 19 - ligne 25; revendications 1,8-10,12 *<br>--- | 1,3-6 | |
| A | EP-A-0 117 473 (BAYER AG)<br>* revendications 1,7-10; exemple 27 *<br>--- | 1,3-6 | |
| A,D | EP-A-0 406 112 (LABORATORIES DEL DR. ESTEVE, S.A.)<br>* le document en entier *<br>--- | 7 | |
| A,D | EP-A-0 394 120 (LABORATORIES DEL DR. ESTEVE, S.A.)<br>* le document en entier *<br>--- | 1 | DOMAINES TECHNIQUES RECHERCHES (Int. Cl.5 ) |
| A,D | EP-A-0 388 298 (LABORATORIES DEL DR. ESTEVE, S.A.)<br>* le document en entier *<br>--- | 1 | C07D<br>A61K |
| A,D | EP-A-0 324 298 (LABORATORIES DEL DR. ESTEVE, S.A.)<br>* le document en entier *<br>----- | 1 | |

Le présent rapport a été établi pour toutes les revendications

| Lieu de la recherche | Date d'achèvement de la recherche | Examinateur |
| --- | --- | --- |
| LA HAYE | 24 AOUT 1992 | P. BOSMA |

**CATEGORIE DES DOCUMENTS CITES**

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P0402)